# EUROPÄISCHE PATENTSCHRIFT

(11) **EP 0 913 396 B1**
(45) Veröffentlichungstag und Bekanntmachung des Hinweises auf die Patenterteilung: **17.09.2003**
(21) Anmeldenummer: 98120379.7
(22) Anmeldetag: 28.10.1998
(51) Int. Cl.: C07D 311/68, A61K 31/35, C07D 311/70

(54) **Sulfonamid-substituierte Benzopyranderivate, Verfahren zu ihrer Herstellung, ihre Verwendung als Medikament sowie sie enthaltende pharmazeutische Zubereitungen**
Sulphonamide substituted benzopyran derivatives, process of preparation, their use as medicines and pharmaceutical compositions containing them
Dérivés de benzopyranne substitués par sulfonamides, procédés pour leur préparation, leur utilisation comme médicaments et compositions pharmaceutiques les contenant

(30) Priorität: 03.11.1997 DE 19748469
(43) Veröffentlichungstag der Anmeldung: 06.05.1999
(73) Patentinhaber: Aventis Pharma Deutschland GmbH, 65929 Frankfurt am Main (DE)
(72) Erfinder: Brendel, Joachim Dr., 61118 Bad Vilbel (DE); Gerlach, Uwe Dr., 65795 Hattersheim (DE); Lang, Hans Jochen Dr., 65719 Hofheim (DE); Weidmann, Klaus Dr., 61476 Kronberg (DE)

(56) Entgegenhaltungen:
- EP-A- 0 807 629
- EP-A- 0 860 440
- BUSCH A E ET AL: "Role of the ISK protein in the IminK channel complex" TRENDS IN PHARMACOLOGICAL SCIENCES,GB,ELSEVIER TRENDS JOURNAL, CAMBRIDGE, Bd. 18, Nr. 1, 1997, Seiten 26-29, XP004049948 ISSN: 0165-6147
- LOHRMANN E ET AL: "A NEW CLASS OF INHIBITORS OF CAMP-MEDIATED CL- SECRETION IN RABBIT COLON, ACTING BY THE REDUCTION OF CAMP-ACTIVATED K+ CONDUCTANCE" PFLUEGERS ARCHIV,DE,SPRINGER VERLAG, BERLIN, Bd. 429, 1995, Seiten 517-530, XP002038768 ISSN: 0031-6768

## Beschreibung

Die Erfindung betrifft Verbindungen der Formel I, 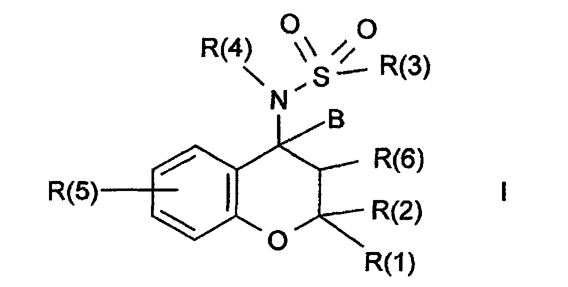 worin R(1), R(2), R(3), R(4), R(5), R(6) und B die im folgenden angegebenen Bedeutungen haben, ihre Herstellung und ihre Verwendung, insbesondere in Arzneimitteln. Die Verbindungen beeinflussen den durch cyclisches Adenosinmonophosphat (cAMP) geöffneten Kaliumkanal bzw. den I_{Ks}-Kanal und eignen sich in hervorragender Weise als Arzneimittelwirkstoffe, beispielsweise zur Prophylaxe und Therapie von Herz-Kreislauferkrankungen, insbesondere Arrhythmien, zur Behandlung von Ulcera des Magen-Darm-Bereichs oder zur Behandlung von Durchfallerkrankungen.

In der Arzneimittelchemie wurde in den letzten Jahren die Klasse der 4-Acyiaminochroman-Derivate intensiv bearbeitet. Prominentester Vertreter dieser Klasse ist das Cromakalim der Formel A (J. Med. Chem. 1986, 29, 2194).

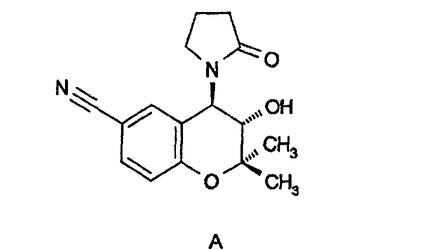

Bei Cromakalim und anderen verwandten 4-Acylaminochroman-Derivaten handelt es sich um Verbindungen mit relaxierender Wirkung auf glattmuskuläre Organe, so daß sie zur Senkung des erhöhten Blutdruckes infolge Gefäßmuskelrelaxation und in der Behandlung des Asthmas infolge der Relaxation der glatten Muskulatur der Atemwege verwendet werden. All diesen Präparaten ist gemeinsam, daß sie auf zellulärer Ebene beispielsweise von glatten Muskelzellen wirken und dort zu einer Öffnung bestimmter ATP-sensitiver K⁺-Kanäle führen. Der durch den Austritt von K⁺-Ionen induzierte Anstieg von negativer Ladung in der Zelle (Hyperpolarisation) wirkt über Sekundärmechanismen der Erhöhung der intrazellulären Ca²⁺-Konzentration und damit einer Zellaktivierung entgegen, die z. B. zu einer Muskelkontraktion führt.

Von diesen Acylamino-Derivaten unterscheiden sich die erfindungsgemäßen Verbindungen der Formel I strukturell unter anderem durch den Ersatz der Acylaminogruppe durch eine Sulfonylaminofunktion. Während Cromakalim (Formel A) und analoge Acylaminoverbindungen als Öffner ATP-sensitiver K⁺-Kanäle wirken, zeigen die erfindungsgemäßen Verbindungen der Formel I mit der Sulfonylaminostruktur jedoch keine öffnende Wirkung auf diesen K⁺(ATP)-Kanal, sondern überraschenderweise eine starke und spezifische blockierende (schließende) Wirkung auf einen K⁺-Kanal, der durch cyclisches Adenosinmonophosphat (cAMP) geöffnet wird und sich grundlegend vom erwähnten K⁺(ATP)-Kanal unterscheidet. Neuere Untersuchungen zeigen, daß dieser an Dickdarmgewebe identifizierte K⁺(cAMP) -Kanal sehr ähnlich, vielleicht sogar identisch, mit dem am Herzmuskel identifizierten I_{Ks}-Kanal ist. In der Tat konnte für die erfindungsgemäßen Verbindungen der Formel I eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP) -Kanals bzw. des I_{Ks}-Kanals entwickeln die erfindungsgemäßen Verbindungen im lebenden Organismus pharmakologische Wirkungen von hoher therapeutischer Verwertbarkeit.

Neben den oben genannten Cromakalim- bzw. Acylaminochroman-Derivaten werden in der Literatur auch Verbindungen mit 4-Sulfonylaminochroman-Struktur beschrieben. In der EP-A-389 861 und der JP 01294677 werden 3-Hydroxychroman- bzw. Chromen-Derivate mit einer cyclischen 4-Sulfonylaminogruppe beschrieben (z.B. Verbindung B), die über eine Aktivierung des K⁺(ATP)-Kanals als Antihypertensiva wirken sollen. In den Ansprüchen der eben erwähnten EP-A-389 861 sind auch Verbindungen mit nicht cyclischen 4-Sulfonylaminoresten umfaßt, die den in dieser Erfindung beschriebenen Verbindungen ähneln, sich jedoch in der Bedeutung von R(5) von diesen unterscheiden. Überraschenderweise war nun gefunden worden, daß gerade die in dieser Anmeldung beschriebenen Substituenten für R(5), insbesondere Alkoxy, z.B. Butoxy oder 4,4,4-Trifluorbutoxy, gegenüber Verbindungen mit den in der EP-A-389 861 aufgeführten Resten erhebliche Vorteile, insbesondere in der Potenz zur Blockierung des I_{Ks}-Kanals, aufweisen. Die von den Autoren der EP-A-389 861 beanspruchten, jedoch nicht mit Beispielen belegten, analogen Verbindungen weisen ebenfalls eine Wirkung auf den I_{Ks}-Kanal auf, die jedoch schwächer ist und von den Autoren dieser Anmeldung nicht erkannt wurde. Wenn auch von den Autoren u. a. eine Anwendung zur Behandlung von Arrhythmien angegeben wird, so ist doch zu sagen, daß die dort beschriebenen Verbindungen, die die Öffnung des K⁺(ATP)-Kanals bewirken sollen, über die Öffnung dieses Kanals zu einer Verkürzung der Repolarisationsdauer führen sollten und damit eher proarrhythmisch wirken sollten. In diesem Zusammenhang sei auf eine grundlegende Arbeit von Lucchesi et al. (J. Cardiovasc. Pharmacol. 15, 1990, 452) hingewiesen, in der eindrucksvoll gezeigt werden konnte, daß K⁺(ATP)-Kanalöffner am suerstoffunterversorgten kranken Herzen oder bei plötzlichen Ischämien nicht antiarrhythmisch wirken, sondern sogar im Gegenteil lebensbedrohliche profibrillatorische Effekte verursachen.

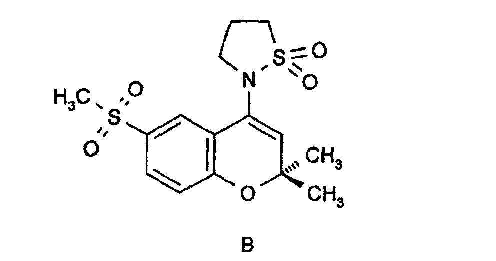

Neben den oben genannten Verbindungen mit 4-Sulfonylaminochroman-Struktur sind noch einige weitere bekannt, die sich aber sowohl in der Struktur als auch in der biologischen Wirkung deutlich von den erfindungsgemäßen Verbindungen der Formel I unterscheiden. So werden in der EP-A-315 009 Chromanderivate mit 4- Phenylsulfonylaminostruktur beschrieben, die sich durch antithrombotische und antiallergische Eigenschaften auszeichnen. In der EP-A-370 901 werden 3-Hydroxychroman- bzw. Chromen-Derivate mit einer 4-Sulfonylaminogruppe, wobei die restliche Valenz des N-Atoms ein Wasserstoffatom trägt, beschrieben, die über ZNS-Wirkungen verfügen. Weitere 4-Sulfonylaminochroman-Derivate werden in Bioorg. Med. Chem. Lett. 4 (1994), 769 - 773: "N-sulfonamides of benzopyranrelated potassium channel openers: conversion of glyburyde insensitive smooth muscle relaxants to potent smooth muscle contractors" sowie in FEBS Letters 396 (1996), 271 - 275: "Specific blockade of slowly activating I_{sK} channels by chromanols ..." und Pflügers Arch. - Eur. J. Physiol. 429 (1995), 517 - 530: "A new class of inhibitors of cAMP-mediated Cl- secretion in rabbit colon, acting by the reduction of cAMP-activated K⁺ conductance" beschrieben.

Die vorliegende Erfindung betrifft Verbindungen der Formel I, 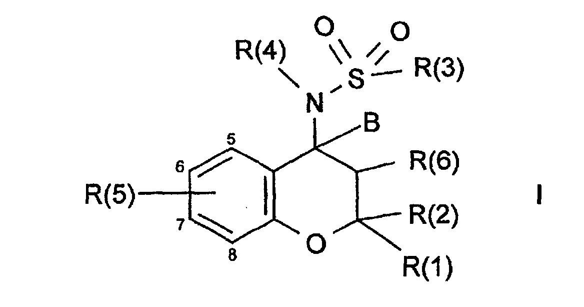 worin R(5) in einer der mit 5, 6, 7 und 8 gekennzeichneten Positionen gebunden ist und worin bedeuten:
R(1) und R(2)
   unabhängig voneinander Wasserstoff, CF₃, C₂F₅, C₃F₇, Alkyl mit 1, 2, 3, 4, 5 oder 6 C-Atomen oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino; oder
R(1) und R(2)
   gemeinsam eine Alkylenkette mit 2, 3, 4, 5, 6, 7, 8, 9 oder 10 C-Atomen;
R(3) R(10)-CₙH₂ₙ-NR(11)- oder R(10)-CₙH₂ₙ-,
   wobei eine CH₂-Gruppe in den Gruppen CₙH₂ₙ ersetzt sein kann durch -O-, -CO-, -S-, -SO-, -SO₂- oder -NR(12a)-; R(12a) Wasserstoff, Methyl oder Ethyl;
   R(10) Wasserstoff, Methyl, Cycloalkyl mit 3, 4, 5, 6, 7 oder 8 C-Atomen, CF₃, C₂F₅ oder C₃F₇;
   n Null, 1, 2, 3, 4, 5, 6, 7, 8, 9 oder 10;
   R(11) Wasserstoff oder Alkyl mit 1, 2, 3, 4, 5 oder 6 C-Atomen;
   oder
   R(10) und R(11) gemeinsam eine Bindung, sofern n nicht kleiner als 3 ist; oder
R(3) gemeinsam mit R(4)
   eine Alkylenkette mit 3, 4, 5, 6, 7 oder 8 C-Atomen,
   wobei eine CH₂-Gruppe der Alkylenkette durch -O-, -CO-, -S-, -SO-, -SO₂- oder -NR(12a)- ersetzt sein kann; R(12a) Wasserstoff, Methyl oder Ethyl;
R(4) R(13)-CᵣH₂ᵣ,
   wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂- oder -NR(14)-;
   R(14) Wasserstoff oder Alkyl mit 1, 2 oder 3 C-Atomen;
   R(13) CH₃, CF₃, C₂F₅, C₃F₇, Cycloalkyl mit 3, 4, 5, 6, 7 oder 8 C-Atomen, NR(15)R(16), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(15) und R(16)
   gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
   r Null, 1, 2, 3, 4, 5, 6, 7, 8, 9, 10, 11, 12, 13, 14, 15, 16, 17, 18, 19 oder 20;
R(5) -Y-CₛH₂ₛ-R(18) oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Y -O-, -S- oder-NR(10c)-; R(10c) Wasserstoff oder Alkyl mit 1, 2 oder 3 C-Atomen;
   s 1, 2, 3, 4, 5, 6, 7 oder 8;
   R(18) Wasserstoff, CF₃, C₂F₅, C₃F₇, Cycloalkyl mit 3, 4, 5, 6, 7 oder 8 C-Atomen, -COOR(21), NR(15a)R(16a), ein unsubstituierter N-haltiger Heterocyclus mit 1, 2, 3, 4, 5, 6, 7, 8 oder 9 C-Atomen, Phenyl oder Thienyl,
   wobei Phenyl und Thienyl unsubstituiert sind oder substituiert mit 1 oder 2 Substituenten, ausgewählt aus der Gruppe bestehend aus F, Cl, Br, I, CF₃, Methyl, Methoxy, Sulfamoyl, Methylsulfonyl und Methylsulfonylamino;
   R(15a) und R(16a)
   gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
   R(21) Wasserstoff oder Alkyl mit 1, 2 oder 3 C-Atomen;
R(6) OR(10d) oder OCOR(10d);
   R(10d) Wasserstoff oder Alkyl mit 1, 2 oder 3 C-Atomen;
B Wasserstoff;
   oder
R(6) und B
   gemeinsam eine Bindung; sowie ihre physiologisch verträglichen Salze.

Bevorzugt sind Verbindungen der Formel I, worin bedeuten
R(1) und R(2)
   unabhängig voneinander Wasserstoff, CF₃, C₂F₅, C₃F₇ oder Alkyl mit 1, 2, 3, 4, 5 oder 6 C-Atomen; oder
R(1) und R(2)
   gemeinsam eine Alkylenkette mit 2, 3, 4, 5, 6, 7, 8, 9 oder 10 C-Atomen;
R(3) R(10)-CₙH₂ₙ;
   R(10) Methyl, CF₃, C₂F₅ oder C₃F₇;
   n Null, 1, 2, 3, 4, 5 oder 6;
R(4) R(13)-CᵣH₂ᵣ,
   wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂- oder -NR(14)-;
   R(14) Wasserstoff oder Alkyl mit 1, 2 oder 3 C-Atomen;
   R(13) CH₃, CF₃, C₂F₅, C₃F₇, Cycloalkyl mit 3, 4, 5, 6, 7 oder 8 C-Atomen, NR(15)R(16), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(15) und R(16) gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
   r Null, 1, 2, 3, 4, 5, 6, 7, 8, 9, 10, 11 oder 12;
R(5) -Y-CₛH₂ₛ-R(18) oder Phenyl,
   das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   Y -O- oder -S-;
   s 1, 2, 3, 4, 5, 6, 7 oder 8;
   R(18) Wasserstoff, CF₃, C₂F₅, C₃F₇, Cycloalkyl mit 3, 4, 5, 6, 7 oder 8 C-Atomen, -COOR(21), NR(15a)R(16a), ein unsubstituierter N-haltiger Heterocyclus mit 1, 2, 3, 4, 5, 6, 7, 8 oder 9 C-Atomen, Phenyl oder Thienyl,
   wobei Phenyl und Thienyl unsubstituiert sind oder substituiert mit 1 oder 2 Substituenten, ausgewählt aus der Gruppe bestehend aus F, Cl, Br, I, CF₃, Methyl, Methoxy, Sulfamoyl, Methylsulfonyl und Methylsulfonylamino;
   R(15a) und R(16a) gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
   R(21) Alkyl mit 1, 2 oder 3 C-Atomen;
R(6) OR(10d) oder OCOR(10d);
   R(10d) Wasserstoff oder Alkyl mit 1, 2 oder 3 C-Atomen;
B Wasserstoff;
   oder
R(6) und B
   gemeinsam eine Bindung; sowie ihre physiologisch verträglichen Salze.

Besonders bevorzugt sind Verbindungen der Formel I, in der R(5) an der mit 6 gekennzeichneten Position gebunden ist, also Verbindungen der Formel Ia, 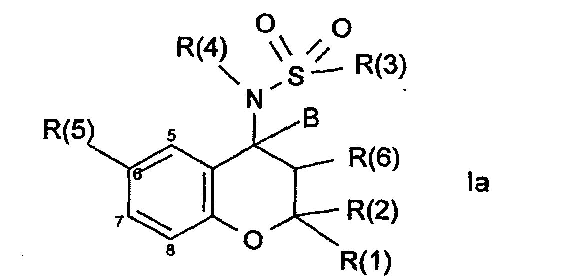 worin die Reste R(1), R(2), R(3), R(4), R(5), R(6) und B die oben als bevorzugt angegebenen Bedeutungen haben.

Ganz besonders bevorzugt sind Verbindungen der Formel I a, worin bedeuten
R(1) und R(2)
   unabhängig voneinander Wasserstoff, CF₃ oder Alkyl mit 1, 2 oder 3 C-Atomen;
   oder
R(1) und R(2)
   gemeinsam eine Alkylenkette mit 2, 3, 4, 5 oder 6 C-Atomen;
R(3) R(10)-CₙH₂ₙ;
   R(10) Methyl oder CF₃;
   n Null, 1 oder 2;
R(4) R(13)-CᵣH₂ᵣ,
   wobei eine CH₂-Gruppe der Gruppe CᵣH₂ᵣ ersetzt sein kann durch -O-, -CO-, -CO-O-, -O-CO-, -S-, -SO-, -SO₂- oder -NR(14)-; R(14) Wasserstoff oder Alkyl mit 1, 2 oder 3 C-Atomen;
   R(13) CH₃, CF₃, NR(15)R(16), Phenyl oder ein N-haltiger Heterocyclus mit 1, 2, 3, 4, 5, 6, 7, 8 oder 9 C-Atomen,
   wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
   R(15) und R(16) gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
   r Null, 1, 2, 3, 4, 5, 6, 7 oder 8;
R(5) -Y-CₛH₂ₛ-R(18);
   Y -O-;
   s 1, 2, 3, 4, 5 oder 6;
   R(18) Wasserstoff, CF₃, -COOR(21), NR(15a)R(16a), ein unsubstituierter N-haltiger Heterocyclus mit 1, 2, 3, 4, 5, 6, 7, 8 oder 9 C-Atomen, Phenyl oder Thienyl,
   wobei Phenyl und Thienyl unsubstituiert sind oder substituiert mit 1 oder 2 Substituenten, ausgewählt aus der Gruppe bestehend aus F, Cl, Br, I, CF₃, Methyl, Methoxy, Sulfamoyl, Methylsulfonyl und Methylsulfonylamino;
   R(15a) und R(16a)
   gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
   R(21) Alkyl mit 1, 2 oder 3 C-Atomen;
   R(6) OH;
B Wasserstoff;
   oder
R(6) und B
   gemeinsam eine Bindung;
   sowie ihre physiologisch verträglichen Salze.

Speziell bevorzugt sind Verbindungen der Formel Ia, worin bedeuten
R(1) und R(2)
   Methyl;
R(3) Methyl oder Ethyl;
R(4) R(13)-CᵣH₂ᵣ,
   wobei eine CH₂-Gruppe der Gruppe CᵣH₂ᵣ ersetzt sein kann durch -O-, -CO-, -CO-O- oder -O-CO;
   R(13) CH₃ oder CF₃;
   r Null, 1, 2, 3, 4, 5, 6, 7 oder 8;
R(5) -Y-CₛH₂ₛ-R(18);
   Y -O-;
   s 1, 2, 3, 4, 5 oder 6;
   R(18) Wasserstoff, CF₃, -COOR(21), Phenyl oder Thienyl,
   wobei Phenyl und Thienyl unsubstituiert sind oder substituiert mit 1 oder 2 Substituenten, ausgewählt aus der Gruppe bestehend aus F, Cl, Br, CF₃, Methyl oder Methoxy;
   R(21) Alkyl mit 1, 2 oder 3 C-Atomen;
R(6) und B
   gemeinsam eine Bindung; sowie ihre physiologisch verträglichen Salze.

Speziell bevorzugt sind auch Verbindungen der Formel Ia, worin bedeuten
R(1) und R(2) Methyl;
R(3) Methyl oder Ethyl;
R(4) R(13)-CᵣH₂ᵣ,
   wobei eine CH₂-Gruppe der Gruppe CᵣH₂ᵣ ersetzt sein kann durch -O-, -CO-, -CO-O- oder -O-CO;
   R(13) CH₃ oder CF₃; r Null, 1, 2, 3, 4, 5, 6, 7 oder 8;
R(5) -Y-CₛH₂ₛ-R(18);
   Y -O-;
   s 1, 2, 3, 4, 5 oder 6;
   R(18) Wasserstoff, CF₃, Phenyl oder Thienyl,
   wobei Phenyl und Thienyl unsubstituiert sind oder substituiert mit 1 oder 2 Substituenten, ausgewählt aus der Gruppe bestehend aus F, Cl, Br, CF₃, Methyl oder Methoxy;
R(6) OH;
B Wasserstoff;
   sowie ihre physiologisch verträglichen Salze.

Alkylreste und Alkylenreste können geradkettig oder verzweigt sein. Dies gilt auch für die Alkylenreste der Formeln CᵣH₂ᵣ, CₙH₂ₙ und CₛH₂ₛ. Alkylreste und Alkylenreste können auch geradkettig oder verzweigt sein, wenn sie substituiert sind oder in anderen Resten enthalten sind, z. B. in einem Alkoxyrest oder in einem Alkylmercaptorest oder in einem fluorierten Alkylrest. Beispiele für Alkylreste sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,4-Butylen, 1,5-Pentylen, 2,2-Dimethyl-1,3-propylen, 1,6-Hexylen, usw. sind Beispiele für Alkylenreste.

Als N-haltige Heterocyclen mit 1, 2, 3, 4, 5, 6, 7, 8 oder 9 C-Atomen gelten insbesondere die aromatischen Systeme 1-, 2- oder 3- Pyrrolyl, 1-, 2-, 4- oder 5-Imidazolyl, 1-, 3-, 4- oder 5-Pyrazolyl, 1,2,3-Triazol-1-, -4- oder 5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder 5-yl, 1,2,4-Oxadiazol-3-oder 5-yl, 1,3,4-Oxadiazol-2-yl oder - 5-yl, 2-, 4- oder 5-Thiazolyl, 3-, 4- oder 5-lsothiazolyl, 1,3,4-Thiadiazol-2- oder -5-yl, 1,2,4-Thiadiazol-3- oder -5-yl, 1,2,3-Thiadiazol-4- oder 5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-lsochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl.

Besonders bevorzugt sind die N-haltigen Heterocyclen Pyrrolyl, Imidazolyl, Chinolyl, Pyrazolyl, Pyridyl, Pyrazinyl, Pyrimidinyl und Pyridazinyl.

Thienyl steht sowohl für 2- als auch 3-Thienyl.

Monosubstituierte Phenylreste können in der 2-, der 3- oder der 4-Position substituiert sein, disubstituierte in der 2,3-, 2,4-, 2,5-, 2,6-, 3,4- oder 3,5-Position. Entsprechendes gilt sinngemäß analog auch für die N-haltigen Heterocylen oder den Thiophenrest.

Bei Disubstitution eines Restes können die Substituenten gleich oder verschieden sein.

Bedeuten die Reste R(1) und R(2) gemeinsam eine Alkylenkette, so bilden diese Reste mit dem sie tragenden Kohlenstoffatom einen Ring, der mit dem 6-Ring in der Formel I ein Kohlenstoffatom gemeinsam hat, es liegt dann also eine Spiroverbindung vor. Bedeuten R(6) und B gemeinsam eine Bindung so liegt ein 2H-Chromengrundgerüst vor. Bedeuten R(10) und R(11) gemeinsam eine Bindung, so steht die Gruppe R(10)-CₙH₂ₙ-NR(11)- bevorzugt für einen über ein Stickstoffatom gebundenen Stickstoffheterocyclus. Wenn R(10) und R(11) gemeinsam eine Bindung bedeuten und die Gruppe R(10)-CₙH₂ₙ-NR(11)- für einen über ein Stickstoffatom gebundenen Stickstoffheterocyclus steht, ist dieser Stickstoffheterocyclus bevorzugt ein 4-Ring oder ein größerer Ring als ein 4-Ring, z. B. ein 5-Ring, 6-Ring oder 7-Ring.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. gehören also in enantiomerenreiner Form, sowohl als links- als auch als rechtsdrehende Antipoden, und auch in Form von Mischungen der beiden Enantiomeren in unterschiedlichen Verhältnissen oder in Form von Racematen zu der Erfindung. Bei Vorliegen einer cis/trans-Isomerie gehören sowohl die cis-Form als auch die trans-Form und Gemische dieser Formen zur Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfaßt die vorliegende Erfindung auch alle tautomeren Formen der Verbindungen der Formel I.

Die Verbindungen der Formel I sind durch unterschiedliche chemische Verfahren herstellbar, die ebenfalls zu der vorliegenden Erfindung gehören. So erhält man beispielsweise eine Verbindung der Formel I, indem man
a) eine Verbindung der Formel II, 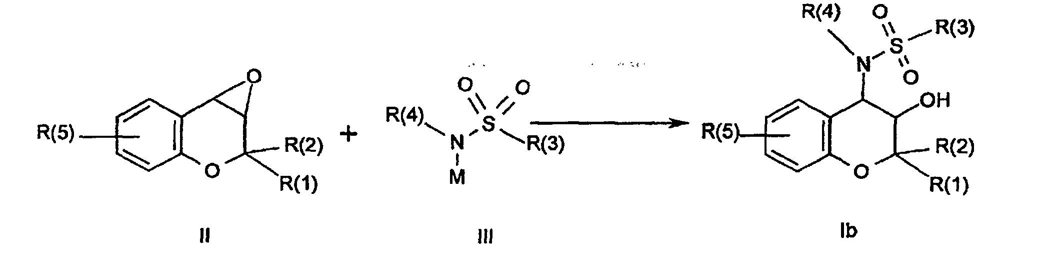 worin R(1), R(2) und R(5) die oben angegebenen Bedeutungen besitzen, mit einem Sulfonamid der Formel III, worin R(3) und R(4) die oben angegebenen Bedeutungen haben und M für Wasserstoff oder ein Metalläquivalent, bevorzugt für Lithium, Natrium oder Kalium, oder M vorteilhaft auch für einen Trialkylsilylrest, z.B. einen Trimethylsilylrest, steht, umsetzt zu einem Chromanol der Formel Ib;
   oder daß man
b) eine Verbindung der Formel I b mit einem Alkylierungsmittel der Formel R(10d)-L oder einem Acylierungsmittel der Formel R(10d)-COL bzw. einem Anhydrid der Formel (R(10d)-CO)₂O, worin R(10d) die oben angegeben Bedeutungen besitzt und L eine nucleofuge Fluchtgruppe, insbesondere F, Cl, Br, I, Methansulfonyloxy oder p-Toluolsulfonyloxy, bedeutet, umsetzt in an sich bekannter Weise im Sinne einer Alkylierungs- bzw. Acylierungsreaktion zu einer Verbindung der Formel I c, worin R(6) OR(10d) oder OCOR(10d) bedeutet; 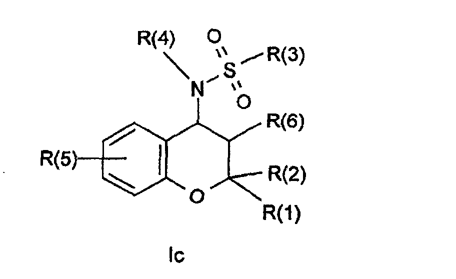 oder daß man
c) eine Verbindung der Formel I b, 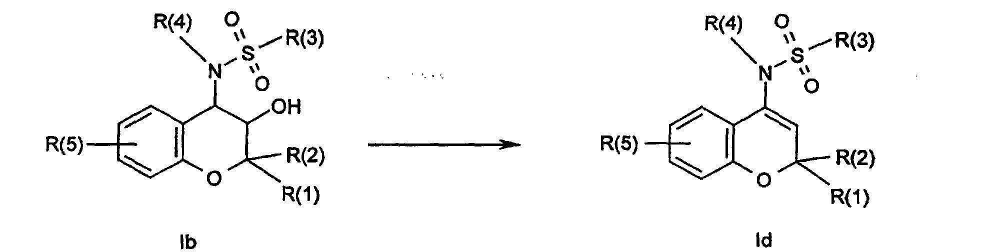 worin R(1), R(2), R(3), R(4) und R(5) die oben angegebenen Bedeutungen haben, im Sinne einer Eliminierungsreaktion zu einer Verbindung der Formel I d, worin R(1), R(2), R(3), R(4) und R(5) die oben angegebenen Bedeutungen haben, umwandelt;
   oder daß man
d) eine Verbindung der Formel IV 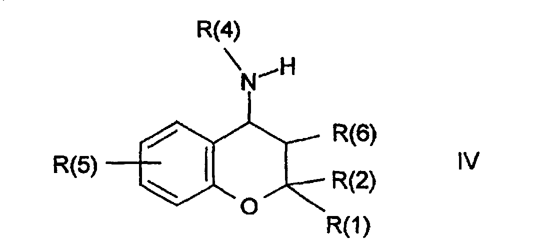 worin R(1), R(2), R(4), R(5) und R(6) die in den Ansprüchen 1 bis 6 angegebenen Bedeutungen besitzen, mit einem Sulfonsäure-Derivat der Formel V 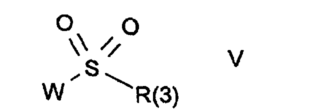 umsetzt, worin R(3) die oben angegebenen Bedeutungen besitzt und W eine nucleofuge Fluchtgruppe, wie z. B. Brom, 1-Imidazolyl, insbesondere aber Chlor, bedeutet;
   oder daß man
e) eine Verbindung der Formel VI 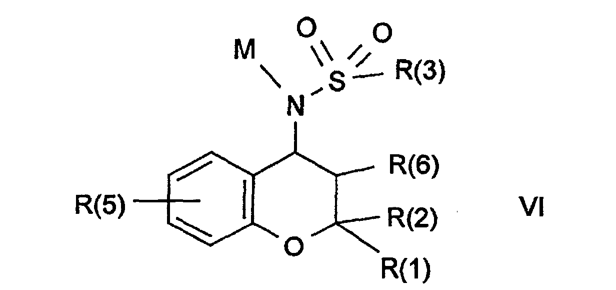 worin R(1), R(2), R(3), R(5) und R(6) die in den Ansprüchen 1 bis 6 angegebenen Bedeutungen besitzen und M für Wasserstoff oder ein Metalläquivalent, bevorzugt für Lithium, Natrium oder Kalium, steht, in an sich bekannter Weise im Sinne einer Alkylierungsreaktion mit einem Alkylierungsmittel der Formel VII umsetzt,

   R(4)-L VII

   worin R(4) und L die oben angegebenen Bedeutungen besitzen;
   oder daß man
f) eine Verbindung der Formel VIII 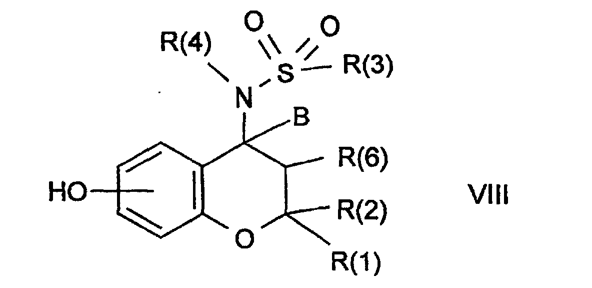 worin R(1), R(2), R(3), R(4), R(6) und B die in den Ansprüchen 1 bis 6 angegebenen Bedeutungen besitzen, mit einer Verbindung der Formel R(18)-CₛH₂ₛ-L, worin R(18), s und L die oben angegebenen Bedeutungen besitzen, umsetzt im Sinne einer Alkylierungsreaktion.

### Verfahrensweise a)

entspricht der nucleophilen Öffnung eines Epoxids der Formel II durch ein Sulfonamid bzw. eines seiner Salze der Formel III. Bei Verwendung eines freien Sulfonamids (Formel III, M = H) ist bevorzugt, aus diesem zunächst durch Einwirkung einer Base ein Sulfonamidsalz (Formel III, M = Metallkation) zu erzeugen, wobei die Deprotonierung des Sulfonamids zum Salz in situ erfolgen kann. Hierzu werden bevorzugt Basen verwendet, die selbst nicht als Nucleophil reagieren, wie z. B. Natriumhydrid, Natriumcarbonat, Kaliumcarbonat, sterisch stark gehinderte Amine, z. B. Dicyclohexylamin, N,N-Dicyclohexyl-ethylamin, oder andere starke Stickstoffbasen mit geringer Nucleophilie, beispielsweise DBU (Diazabicycloundecen), N,N',N'''-Triisopropylguanidin etc. Es können allerdings auch andere üblich verwendete Basen für die Reaktion eingesetzt werden, wie Kalium-tert-butylat, Natriummethylat, Alkalihydrogencarbonate, Alkalihydroxide, wie beispielsweise LiOH, NaOH oder KOH, oder Erdalkalihydroxide, wie beispielsweise Ca(OH)₂.

Die Base kann in stöchiometrischer Menge oder auch katalytisch eingesetzt werden. Als besonders vorteilhaft hat sich die Verwendung des freien Sulfonamids in Gegenwart einer unterstöchiometirschen Menge, z. B. 20 - 70 %, einer geeigneten Base, z. B. Natriumhydrid, erwiesen

Die Reaktion wird vorzugsweise in einem Lösungsmittel durchgeführt, besonders bevorzugt in polaren organischen Lösungsmitteln wie z. B. Dimethylformamid (DMF), Dimethylacetamid (DMA), Dimethylsulfoxid (DMSO), Tetramethylharnstoff (TMU), Hexamethylphosphorsäuretriamid (HMPT), Tetrahydrofuran (THF), Dimethoxyethan (DME) oder anderen Ethern, oder beispielsweise auch in einem Kohlenwasserstoff wie Toluol oder in einem halogenierten Kohlenwasserstoff wie Chloroform oder Methylenchlorid usw. Es kann aber auch in polaren protischen Lösungsmitteln gearbeitet werden, wie z. B. in Wasser, Methanol, Ethanol, Isopropanol, Ethylenglykol oder dessen Oligomeren und deren entsprechenden Halbethem oder auch deren Ethem. Die Reaktion kann auch in Gemischen dieser Lösungsmittel durchgeführt werden. Ebenso kann die Reaktion aber auch ganz bevorzugt ohne Lösungsmittel durchgeführt werden. Die Reaktion wird bevorzugt in einem Temperaturbereich von -10 bis +140 °C, besonders bevorzugt im Bereich von 20 bis 100 °C durchgeführt.

Eine andere bevorzugte Verfahrensweise zur Durchführung dieser Reaktion beinhaltet die Verwendung von Sulfonamidderivaten der Formel III, bei denen M für einen Trialkylsilyl-, z.B. einen Trimethylsilylrest steht. Hierbei ist es vorteilhaft, die Reaktion in Gegenwart eines Fluorids, z.B. Tetrabutylammoniumfluorid, durchzuführen.

Die Epoxide der Formel II erhält man nach literaturbekannten Methoden aus den entsprechenden Olefinen der Formel IX, 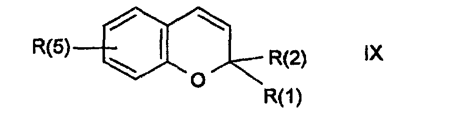 worin R(1), R(2) und R(5) die in den Ansprüchen 1 bis 6 angegebenen Bedeutungen besitzen, z. B. durch Einwirkung eines geeigneten anorganischen oder organischen Peroxids, wie beispielsweise H₂O₂ oder m-Chlorperbenzoesäure, oder durch basenkatalysierte Cyclisierung des entsprechenden Bromhydrins, das aus IX z. B. durch Umsetzung mit N-Bromsuccinimid und Wasser erhalten werden kann. Die Olefine der Formel IX lassen sich erhalten entweder aus den Ketonen der Formel X durch Reduktion der Carbonylgruppe zu einer OH-Funktion und anschließende säurekatalysierte Eliminierung oder durch thermische Cyclisierung von geeignet substituierten Aryl-propargylethem, wie z.B. in J. Org. Chem. 38 (1973) 3832 beschrieben.

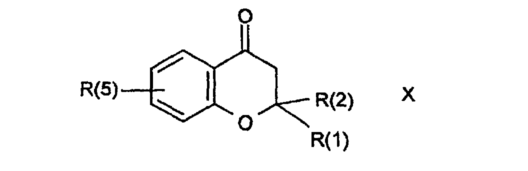

### Verfahrensweise b)

beschreibt die Umwandlung von erfindungsgemäßen Verbindungen der Formel Ib zu anderen erfindungsgemäßen Verbindungen der Formel Ic durch Alkylierung bzw. Acylierung der 3-Hydroxygruppe. Für die Alkylierung wird der Alkohol zunächst durch Einwirkung einer geeigneten Base, wie z.B. Natriumhydrid, in ein Alkoholatsalz überführt, das dann in einem geeigneten polaren Lösungsmittel, wie z.B. Dimethylformamid, bei Temperaturen zwischen 20 und 150°C mit dem Alkylierungsmittel der Formel R(10d)-L umgesetzt wird. Die Deprotonierung des Alkohols zum Salz kann auch in situ erfolgen, wobei dann bevorzugt Basen eingesetzt werden, die selbst nicht alkyliert werden, wie z.B. Kaliumcarbonat. Als weitere geeignete Basen und Lösungsmittel können auch die bereits unter Verfahrensweise a) genannten verwendet werden. Die Acylierung der Verbindungen der Formel Ib erfolgt bevorzugt durch Umsetzung mit dem entsprechenden Anhydrid der Formel (R(10d)-CO)₂O in einem geeigneten polaren Lösungsmittel wie Pyridin oder Dimethylformamid und gegebenenfalls unter Zusatz eines Acylierungskatalysators wie z.B. Dimethylaminopyridin.

### Verfahrensweise c)

beschreibt die Überführung eines Chromanols der Formel Ib in ein Chromen der Formel Id durch Eliminierung. Hierzu kann das Chromanol entweder direkt in Gegenwart einer Säure oder Base einer Wasserabspaltung unterworfen werden oder es kann zunächst eine Aktivierung der Hydroxygruppe erfolgen, z.B. durch Acetylierung mit Acetanhydrid (siehe Verfahrensweise b) oder Mesylierung mit Methansulfonsäurechlorid, woraufhin anschließend eine basenkatalysierte Eliminierung erfolgen kann, z.B. durch Erhitzen mit DBU (Diazabicycloundecen).

### Verfahrensweise d)

beschreibt die an sich bekannte und häufig angewandte Reaktion einer reaktiven Sulfonylverbindung der Formel V, insbesondere einer Chlorsulfonylverbindung (W = Cl), mit einem Amino-Derivat der Formel IV zum entsprechenden Sulfonamid-Derivat der Formel I. Die Reaktion kann prinzipiell ohne Lösungsmittel durchgeführt werden, jedoch werden derartige Reaktionen in den meisten Fällen unter Verwendung eines Lösungsmittels durchgeführt. Die Reaktionsführung geschieht vorzugsweise unter Verwendung eines polaren Lösungsmittels vorzugsweise in Gegenwart einer Base, die selbst vorteilhaft als Lösungsmittel verwendet werden kann, z.B. bei Verwendung von Triethylamin, insbesondere von Pyridin und dessen Homologen. Ebenfalls verwendete Lösungsmittel sind beispielsweise Wasser, aliphatische Alkohole, z.B. Methanol, Ethanol, Isopropanol, sek. Butanol, Ethylenglykol und dessen monomere und oligomere Monoalkyl- und Dialkylether, Tetrahydrofuran, Dioxan, dialkylierte Amide wie DMF, DMA, sowie TMU und HMPT. Man arbeitet dabei bei einer Temperatur von 0 bis 160°C, vorzugsweise von 20 bis 100°C.

Die Amine der Formel IV erhält man in literaturbekannter Weise bevorzugt aus den Epoxiden der Formel II durch nucleophile Öffnung mit den entsprechenden Aminen der Formel R(4)-NH₂ analog der unter a) beschriebenen Verfahrensweise.

### Verfahrensweise e)

repräsentiert die an sich bekannte Alkylierungsreaktion eines Sulfonamids bzw. eines seiner Salze VI mit einem Alkylierungsmittel der Formel VII. Hierzu wird das Sulfonamid zunächst in eines seiner Salze überführt, wofür die in Verfahrensweise a) aufgeführten Basen und Lösungsmittel geeignet sind, das dann bei einer Temperatur zwischen 15 und 150°C mit dem Alkylierungsmittel der Formel VII umgesetzt wird.

Die Sulfonamide der Formel VI erhält man in literaturbekannter Weise bevorzugt aus den Epoxiden der Formel II durch nucleophile Öffnung mit den entsprechenden Sulfonamiden der Formel R(3)-SO₂NH₂ analog der unter a) beschriebenen Verfahrensweise, wobei es hier allerdings vorteilhaft ist, eine stöchiometrische Menge an Base einzusetzen.

### Verfahrensweise f)

beschreibt die Alkylierung eines Phenols der Formel VIII mit einem Alkylierungsmittel der Formel R(18)-CₛH₂ₛ-L. Hierzu wird das Phenol zunächst durch Einwirkung einer geeigneten Base, wie z. B. Natriumhydrid oder einer Phosphazenbase, in ein Phenolatsalz überführt, das dann in einem geeigneten polaren Lösungsmittel, wie z.B. Dimethylformamid oder Dimethylacetamid, bei Temperaturen zwischen 20 und 150°C mit dem Alkylierungsmittel umgesetzt wird. Die Deprotonierung des Alkohols zum Salz kann auch in situ erfolgen, wobei dann bevorzugt Basen eingesetzt werden, die selbst nicht alkyliert werden, wie z.B. Kaliumcarbonat. Als weitere geeignete Basen und Lösungsmittel können auch die bereits unter Verfahrensweise a) genannten verwendet werden.
Die Phenole der Formel VIII erhält man nach den unter a) bis e) beschriebenen Methoden, wobei dann jedoch R(5) jeweils OH bzw. OR (R=geeignete Schutzgruppe, z.B. Benzyl) bedeutet und im letzteren Fall noch eine anschließende Abspaltung der Schutzgruppe erfolgt.

Bei allen Verfahrensweisen kann es angebracht sein, bei bestimmten Reaktionsschritten funktionelle Gruppen im Molekül zeitweilig zu schützen. Solche Schutzgruppentechniken sind dem Fachmann geläufig. Die Auswahl einer Schutzgruppe für in Betracht kommende Gruppen und die Verfahren zu ihrer Einführung und Abspaltung sind in der Literatur beschrieben und können gegebenenfalls ohne Schwierigkeiten dem Einzelfall angepaßt werden.

Es wurde bereits gesagt, daß die Verbindungen der Formel I überraschenderweise eine starke und spezifische blockierende (schließende) Wirkung auf einen K⁺-Kanal haben, der durch cyclisches Adenosinmonophosphat (cAMP) geöffnet wird und sich grundlegend vom wohlbekannten K⁺(ATP)-Kanal unterscheidet, und daß dieser an Dickdarmgewebe identifizierte K⁺(cAMP)-Kanal sehr ähnlich, vielleicht sogar identisch, mit dem am Herzmuskel identifizierten I_{Ks}-Kanal ist. Für die erfindungsgemäßen Verbindungen konnte eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP)-Kanals bzw. des I_{Ks}-Kanals entwickeln die erfindungsgemäßen Verbindungen im lebenden Organismus pharmakologische Wirkungen von hoher therapeutischer Verwertbarkeit und eignen sich in hervorragender Weise als Arzneimittelwirkstoffe für die Therapie und Prophylaxe verschiedener Krankheitsbilder.

So zeichnen sich die erfindungsgemäßen Verbindungen der Formel I als neue Wirkstoffklasse potenter Inhibitoren der stimulierten Magensäuresekretion aus. Die Verbindungen der Formel I sind somit wertvolle Arzneimittelwirkstoffe zur Therapie und Prophylaxe von Ulcera des Magens und des intestinalen Bereiches, beispielsweise des Duodenums. Sie eignen sich ebenfalls infolge ihrer starken magensaftsekretionshemmenden Wirkung als ausgezeichnete Therapeutika zur Therapie und Prophylaxe der Refluxösophagitis.

Die erfindungsgemäßen Verbindungen der Formel I zeichnen sich weiterhin durch eine antidiarrhoische Wirkung aus und sind deshalb als Arzneimittelwirkstoffe zur Therapie und Prophylaxe von Durchfallerkrankungen geeignet.

Weiterhin eignen sich die erfindungsgemäßen Verbindungen der Formel I als Arzneimittelwirkstoffe zur Therapie und Prophylaxe von Herz-Kreislauferkrankungen. Insbesondere können sie zur Therapie und Prophylaxe aller Typen von Arrhythmien verwendet werden, einschließlich atrialer, ventrikulärer und supraventrikulärer Arrhythmien, vor allem von Herzrhythmusstörungen, die durch Aktionspotential-Verlängerung behoben werden können. Sie können speziell angewandt werden zur Therapie und Prophylaxe von atrialer Fibrillation (Vorhofflimmern) und atrialem Flattern (Vorhofflattern) sowie zur Therapie und Prophylaxe von Reentry-Arrhythmien und zur Verhinderung des plötzlichen Herztodes infolge von Kammerflimmern.

Obwohl bereits zahlreiche antiarrhythmisch wirkende Substanzen auf dem Markt sind, gibt es doch keine Verbindung, die hinsichtlich Wirksamkeit, Anwendungsbreite und Nebenwirkungsprofil wirklich zufriedenstellend ist, so daß weiterhin eine Notwendigkeit zur Entwicklung verbesserter Antiarrhythmika besteht.
Die Wirkung zahlreicher bekannter Antiarrhythmika der sogenannten Klasse III beruht auf einer Erhöhung der myocardialen Refraktärzeit durch Verlängerung der Aktionspotentialdauer. Diese wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" I_{K} zugeschrieben, von dem zwei Subtypen existieren, ein schnell aktivierter I_{Kr} und ein langsam aktivierter I_{Ks}. Die meisten bekannten Klasse III-Antiarrhythmika blockieren überwiegend oder ausschließlich I_{Kr} (z.B. Dofetilid, d-Sotalol). Es hat sich jedoch gezeigt, daß diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D.M. Roden; "Current Status of Class III Antiarrhythmic Drug Therapy"; Am. J. Cardiol. 72 (1993), 44B-49B). Bei höheren Herzfrequenzen bzw. Stimulation der β-Rezeptoren hingegen ist die das Aktionspotential verlängernde Wirkung der I_{Kr}-Blocker deutlich reduziert, was darauf zurückgeführt wird, daß unter diesen Bedingungen der I_{Ks} stärker zur Repolarisierung beiträgt. Aus diesen Gründen weisen die erfindungsgemäßen Substanzen, die als I_{Ks}-Blocker wirken, wesentliche Vorteile auf gegenüber den bekannten I_{Kr}-Blockern. Inzwischen wurde auch beschrieben, daß eine Korrelation zwischen I_{Ks}-Kanal-inhibitorischer Wirkung und dem Unterbinden von lebensbedrohlichen cardialen Arrhythmien besteht, wie sie beispielsweise durch β-adrenerge Hyperstimulation ausgelöst werden (z. B. T.J. Colatsky, C.H. Follmer und C.F. Starmer; "Channel Specificity in Antiarrhythmic Drug Action; Mechanism of potassium channel block and its role in suppressing and aggravating cardiac arrhythmias"; Circulation 82 (1990), 2235 - 2242; A.E. Busch, K. Malloy, W.J. Groh, M.D. Varnum, J.P. Adelman und J. Maylie; "The novel class III antiarrhythmics NE-10064 and NE-10133 inhibit I_{sK} channels in xenopus oocytes and I_{Ks} in guinea pig cardiac myocytes"; Biochem. Biophys. Res. Commun. 202 (1994), 265 - 270).

Darüber hinaus tragen die Verbindungen zu einer deutlichen Verbesserung der Herzinsuffizienz, insbesondere der Stauungsherzinsuffizienz (Congestive Heart Failure) bei, vorteilhafterweise in der Kombination mit kontraktionsfördernden (positiv inotropen) Wirkstoffen, z. B. Phosphordiesterasehemmern.

Trotz der therapeutisch nutzbaren Vorteile, die durch eine Blockade des I_{Ks} erzielt werden können, sind bisher nur sehr wenige Verbindungen beschrieben, die diesen Subtyp des "delayed rectifiers" hemmen. Die in der Entwicklung befindliche Substanz Azimilid weist zwar auch eine blockierende Wirkung auf den I_{Ks} auf, blockiert jedoch vorwiegend den I_{Kr} (Selektivität 1 : 10). In der WO-A-95/14470 wird die Verwendung von Benzodiazepinen als selektive Blocker des I_{Ks} beansprucht. Weitere I_{Ks}-Blocker sind beschrieben in FEBS Letters 396 (1996), 271-275: "Specific blockade of slowly activating I_{sK} channels by chromanols..." und Pflügers Arch. - Eur. J. Physiol. 429 (1995), 517-530: "A new class of inhibitors of cAMP-mediated Cl-secretion in rabbit colon, acting by the reduction of cAMP-activated K⁺ conductance". Die Potenz der dort genannten 3-Hydroxychromanole ist jedoch geringer als die der erfindungsgemäßen Verbindungen der Formel I.

Die erfindungsgemäßen Verbindungen der Formel I und ihre physiologisch verträglichen Salze können somit am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Verwendung zur Herstellung von Medikamenten dafür und von Medikamenten mit K⁺-Kanal-blockierender Wirkung. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen, pharmazeutisch einwandfreien Träger- und Hilfsstoffen enthalten. Die pharmazeutischen Zubereitungen enthalten normalerweise 0,1 bis 90 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Die Verbindungen der Formel I können zur Erzielung einer vorteilhaften therapeutischen Wirkung auch mit anderen Arzneiwirkstoffen kombiniert werden. So sind in der Behandlung von Herz-Kreislauferkrankungen vorteilhafte Kombinationen mit herz-kreislaufaktiven Stoffen möglich. Als derartige, für Herz-Kreislauferkrankungen vorteilhafte Kombinationspartner kommen beispielsweise andere Antiarrhythmika, so Klasse I-, Klasse II- oder Klasse III-Antiarrhythmika, in Frage, wie beispielsweise I_{Kr}-Kanalblocker, z.B. Dofetilid, oder weiterhin blutdrucksenkende Stoffe wie ACE-Inhibitoren (beispielsweise Enalapril, Captopril, Ramipril), Angiotensin-Antagonisten, K⁺-Kanalaktivatoren, sowie alpha- und beta-Rezeptorenblocker, aber auch sympathomimetische und adrenerg wirkende Verbindungen, sowie Na⁺/H⁺-Austausch-Inhibitoren, Calciumkanalantagonisten, Phosphodiesterasehemmer und andere positiv inotrop wirkende Stoffe, wie z. B. Digitalisglykoside, oder Diuretika. Weiterhin sind Kombinationen mit antibiotisch wirkenden Substanzen und mit Antiulkusmitteln vorteilhaft, beispielsweise mit H₂-Antagonisten (z. B. Ranitidin, Cimetidin, Famotidin, etc.), insbesondere bei der Anwendung zur Behandlung von Magen-Darmerkrankungen.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wäßrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I und ihre physiologisch verträglichen Salze können auch lyophilisiert werden und die erhaltenen Lyophilisate z. B. zur Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. Üblicherweise beträgt die tägliche Dosis einer Verbindung der Formel I bei Verabreichung an einem etwa 75 kg schweren Patienten 0.001 mg/kg Körpergewicht bis 100 mg/kg Körpergewicht, bevorzugt 0.01 mg/kg Körpergewicht bis 20 mg/kg Körpergewicht. Die Dosis kann in Form einer Einzeldosis verabreicht werden oder in mehrere, z. B. zwei, drei oder vier Einzeldosen aufgeteilt werden. Insbesondere bei der Behandlung akuter Fälle von Herzrhythmusstörungen, beispielsweise auf einer Intensivstation, kann auch eine parenterale Verabreichung durch Injektion oder Infusion, z. B. durch eine intravenöse Dauerinfusion, vorteilhaft sein.

Die Verbindungen der Formel I können auch, wie bereits oben erwähnt, als Zwischenprodukte zur Herstellung weiterer Arzneimittelwirkstoffe eingesetzt werden.

### Experimenteller Teil

### Liste der Abkürzungen

- DBU: 1,8-Diazabicyclo[5.4.0]undec-7-en
- DMF: N,N-Dimethylformamid
- DMSO: Dimethylsulfoxid
- EE: Essigsäureethylester

- F.p.: Schmelzpunkt (Wenn nicht anders angegeben sind die Schmelzpunkte der ungereinigten Rohprodukte angegeben; die Schmelzpunkte der jeweiligen Reinsubstanzen können durchaus deutlich höher liegen)
- i. Vak.: im Vakuum
- LM: Lösungsmittel
- NBS: N-Bromsuccinimid
- RT: Raumtemperatur
- THF: Tetrahydrofuran

### Beispiel 1: (±)-trans-N-(6-Benzyloxy-3-hydroxy-2,2-dimethyl-chroman-4-yl)-N-methylmethansulfonamid

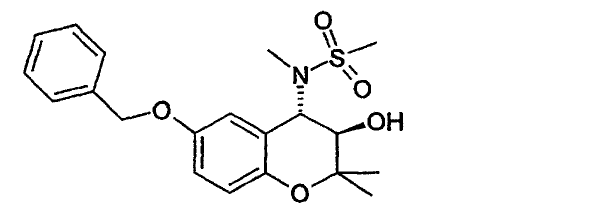

### a) 2,2-Dimethyl-6-hydroxychroman-4-on

Eine Reaktionsmischung aus 100 g (0,65 mol) 2,5-Dihydroxycetophenon in 1 I Acetonitril, 130 ml (1,55 mol) Pyrrolidin und 290 ml (3,95 mol) Aceton wurde 8 h auf 45°C erhitzt. Dann wurden die LM i. Vak. abgezogen und der Rückstand in 1 I EE gelöst. Die organische Phase wurde 2mal mit verdünnter Salzsäure gewaschen, mit Aktivkohle verrührt und über Magnesiumsulfat getrocknet und weitgehend eingeengt. Nach Verrühren des Rückstandes mit Petrolether und Absaugen des Niederschlags wurden 102 g 2,2-Dimethyl-6-hydroxychroman-4-on, F.p. 161°C, erhalten.

### b) 6-Benzyloxy-2,2-dimethylchroman-4-on

25,2 g (131,2 mmol) 6-Hydroxy-2,2-dimethylchroman-4-on wurden bei RT unter Rühren in 350 ml Diethylketon eingetragen und nach Zugabe von 18,0 g (131 mmol) gepulvertem Kaliumcarbonat 30 min bei 75°C gerührt. Nach Abkühlen auf 60°C wurden 15,7 ml (131 mmol) Benzylbromid zugetropft, nach 2 h i.Vak. eingeengt, der Rückstand mit Wasser behandelt und der Feststoff abgesaugt, 37 g, F.p. 109-110 °C.

### c) 6-Benzyloxy-2,2-dimethylchroman-4-ol

Eine Lösung von 20,0 g (71 mmol) 6-Benzyloxy-2,2-dimethylchroman-4-on und 2,94 g (78 mmol) Natriumborhydrid in 100 ml Methanol und 300 ml Ethanol wurde 3 h bei RT gerührt. Dann wurde die Reaktionsmischung auf 1300 ml Eiswasser gegossen und der ausgefallene Niederschlag abgesaugt und im Vakuum getrocknet. Man erhielt 19,3 g 6-Benzyloxy-2,2-dimethylchroman-4-ol, F.p. 83 - 84°C.

### d) 6-Benzyloxy-2,2-dimethyl-2H-chromen

Eine Lösung von 9,6 g (33,8 mmol) 6-Benzyloxy-2,2-dimethylchroman-4-ol und 0,2 g p-Toluolsulfonsäure in 85 ml Toluol wurde 1 h am Wasserabscheider unter Rückfluß erhitzt. Nach dem Abkühlen wurde 2mal mit Natriumhydrogencarbonatlösung gewaschen, über Magnesiumsulfat getrocknet und i. Vak. eingeengt, und man erhielt 7,6 g 6-Benzyloxy-2,2-dimethyl-2H-chromen.

### e) 6-Benzyloxy-3-brom-2,2-dimethyl-chroman-4-ol

Zu einer Lösung von 7,5 g (28, 2 mmol) 6-Benzyloxy-2,2-dimethyl-2H-chromen in 108 ml DMSO und 0,9 ml (48,7 mmol) Wasser wurden unter gutem Rühren 5,05 g (28,3 mmol) NBS auf einmal zugegeben und über Nacht bei RT weiter gerührt. Die Reaktionsmischung wurde auf 450 ml Wasser gegossen, 1 h nachgerührt, der Niederschlag abgesaugt, mit Wasser gewaschen und im Vakuum getrocknet. Man erhielt 9,5 g 6-Benzyloxy-3-brom-2,2-dimethyl-chroman-4-ol, F.p. 126 - 128°C.

### f) 6-Benzyloxy-2,2-dimethyl-3,4-epoxy-chroman

Eine Lösung von 9,5 g (28,5 mmol) 6-Benzyloxy-3-brom-2,2-dimethyl-chroman-4-ol in 100 ml THF wurde über Nacht mit 4,6 g (82 mmol) Kaliumhydroxid-Pulver gerührt. Anschließend wurde der Ansatz über Celite filtriert, das Filtrat einrotiert und man erhielt 8,3 g 6-Benzyloxy-2,2-dimethyl-3,4-epoxy-chroman, F.p. 70 - 72°C.

### g) N-(6-Benzyloxy-3-hydroxy-2,2-dimethyl-chroman-4-yl)-N-methylmethansulfonamid

Zu einer Suspension von 0,21 g (7 mmol) 80 proz. Natriumhydrid in 15 ml DMSO wurden 4,25 g (39 mmol) Methansulfonsäuremethylamid in 7,5 ml DMSO zugetropft und es wurde 30 min bei RT gerührt. Dann wurden 8,2 g (29 mmol) 6-Benzyloxy-2,2-dimethyl-3,4-epoxy-chroman, gelöst in 18 ml DMSO, zugetropft, und der Ansatz wurde 2 Tage auf 50 °C erwärmt. Anschließend wurde auf Wasser gegossen, der Niederschlag abgesaugt, im Vakuum gut getrocknet, und man erhielt 8,8 g N-(6-Benzyloxy-3-hydroxy-2,2-dimethyl-chroman-4-yl)-N-methyl-methansulfonamid, F.p. 162 - 164°C.

### Beispiel 2: (±)-trans-N-(6-Benzyloxy-3-acetoxy-2,2-dimethyl-chroman-4-yl)-N-methylmethansulfonamid

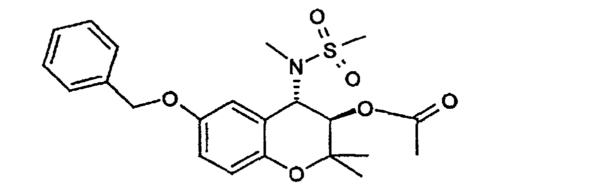

Eine Lösung von 4,5 g N-(6-Benzyloxy-3-hydroxy-2,2-dimethyl-chroman-4-yl)-N-methyl-methansulfonamid und 37 ml Acetanhydrid in 74 ml Pyridin wurde über Nacht bei RT stehen lassen. Der Ansatz wurde im Vakuum eingeengt und der Rückstand in EE gelöst, nacheinander mit verd. Salzsäure und gesättigter Natriumbicarbonatlösung gewaschen und über Magnesiumsulfat getrocknet. Nach Abziehen des Lösungsmittels erhielt man 4,7 g N-(6-Benzyloxy-3-acetoxy-2,2-dimethylchroman-4-yl)-N-methyl-methansulfonamid, F.p. 124 125°C.

### Beispiel 3: (±)-trans-N-(6-Benzyloxy-3-methoxy-2,2-dimethyl-chroman-4-yl)-N-methyl-methansulfonamid

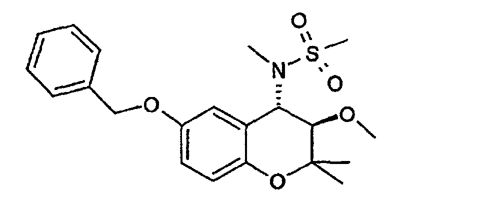

0,5 g (1,3 mmol) N-(6-Benzyloxy-3-hydroxy-2,2-dimethyl-chroman-4-yl)-N-methylmethansulfonamid (Beispiel 1g) gelöst in 4 ml DMF wurden zu einer Suspension von 0,05 g (1,7 mmol) Natriumhydrid in 3 ml DMF getropft. Nach 30 min Rühren bei RT wurden 0,25 g (1,8 mmol) Methyliodid zugegeben und 3 h bei RT nachgerührt. Die Reaktionsmischung wurde im Vakuum eingeengt, der Rückstand in Wasser und EE aufgenommen, und die organische Phase wurde mit verd. Salzsäure und gesättigter Natriumbicarbonatlösung gewaschen. Nach Trocknen über Magnesiumsulfat und Einengen im Vakuum wurden 0,52 g N-(6-Benzyloxy-3-methoxy-2,2-dimethylchroman-4-yl)-N-methyl-methansulfonamid erhalten, F.p. 119 - 121°C.

### Beispiel 4: N-(6-Benzyloxy-2,2-dimethyl-2H-chromen-4-yl)-N-methylmethansulfonamid

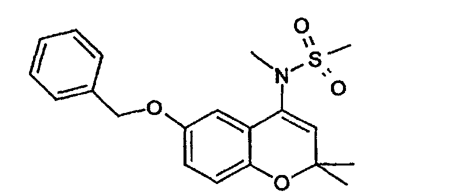

Eine Lösung von 1,0 g (2,3 mmol) N-(6-Benzyloxy-3-acetoxy-2,2-dimethyl-chroman-4-yl)-N-methyl-methansulfonamid (Beispiel 2) und 2,1 g (13,8 mmol) DBU in 4,2 ml Toluol wurde 5 h auf 105°C erhitzt. Die Reaktionsmischung wurde mit EE verdünnt und mit Salzsäure gewaschen, bis zur sauren Reaktion der wäßrigen Phase. Nach Waschen mit Natriumbicarbonatlösung, Trocknen über Magnesiumsulfat und Einengen i. Vak. erhielt man 0,85 g N-(6-Benzyloxy-2,2-dimethyl-2H-chromen-4-yl)-N-methyl-methansulfonamid.

### Beispiel 5: (±)-trans-N-(6-Butoxy-3-hydroxy-2,2-dimethyl-chroman-4-yl)-N-methylmethansulfonamid

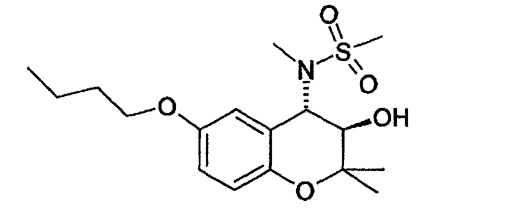

### a) 6-Butoxy-2,2-dimethylchroman-4-on

Zu einer Suspension von 9,0 g (0,3 mol) 80proz. Natriumhydrid in 500 ml DMF wurde eine Lösung von 50 g (0,26 mol) 2,2-Dimethyl-6-hydroxychroman-4-on (Beispiel 1a) in 500 ml DMF zugetropft. Nach 90 min Rühren bei RT wurden 49 g (0,265 mol) lodbutan zugegeben und weitere 90 min bei RT gerührt. Dann wurde die Reaktionsmischung im Vakuum eingeengt, der Rückstand mit Wasser versetzt und mehrmals mit EE extrahiert. Die organischen Phasen wurden mit 5 M Natronlauge gewaschen, mit Aktivkohle und Magnesiumsulfat gerührt, filtriert und eingeengt. Man erhielt 57,6 g 6-Butoxy-2,2-dimethylchroman-4-on.

### b) 6-Butoxy-2,2-dimethyl-2H-chromen

wurde erhalten aus 6-Butoxy-2,2-dimethylchroman-4-on analog der in Beispiel 1c und 1d beschriebenen Verfahrensweise.
Alternativ wurde die Verbindung auch auf folgendem Wege erhalten:
Zu einer Lösung von 25 g 4-Butoxyphenol (150 mmol) in 350 ml Toluol wurden zunächst 4,5 g (150 mmol) 80 proz. Natriumhydrid und nach 15 min eine Lösung von 23 g (224 mmol) 3-Chlor-3-methyl-butin bei RT zugetropft und dann wurde 10 h unter Rückfluß erhitzt. Nach dem Abkühlen wurde mit 5 M Natronlauge und Wasser gewaschen, im Vakuum eingeengt und durch Chromatographie an Kieselgel mit Cyclohexan / EE 9 : 1 gereinigt.

### c) 6-Butoxy-3-brom-2,2-dimethyl-chroman-4-ol

wurde erhalten aus 6-Butoxy-2,2-dimethyl-2H-chromen analog der in Beispiel 1e beschriebenen Verfahrensweise; F.p. 72 - 74°C.

### d) 6-Butoxy-2,2-dimethyl-3,4-epoxy-chroman

Aus 1,0 g (3 mmol) 6-Butoxy-3-brom-2,2-dimethyl-chroman-4-ol wurden analog Beispiel 1f 0,8 g 6-Butoxy-2,2-dimethyl-3,4-epoxy-chroman als Öl erhalten.

### e) N-(6-Butoxy-3-hydroxy-2,2-dimethyl-chroman-4-yl)-N-methyl-methansulfonamid

Zu einer Suspension von 0,02 g (0,7 mmol) 80 proz. Natriumhydrid in 2 ml DMSO wurden 0,47 g (4,3 mmol) Methansulfonsäuremethylamid in 1 ml DMSO zugetropft und es wurde 30 min bei RT gerührt. Dann wurden 0,8 g (3,2 mmol) 6-Butoxy-2,2-dimethyl-3,4-epoxy-chroman, gelöst in 1 ml DMSO, zugetropft, und der Ansatz wurde 5 Tage bei RT stehen gelassen und dann noch 9 h auf 50 °C erwärmt. Anschließend wurde auf Wasser gegossen, der Niederschlag abgesaugt, im Vakuum gut getrocknet, und man erhielt 0,82 g N-(6-Butoxy-3-hydroxy-2,2-dimethylchroman-4-yl)-N-methyl-methansulfonamid, F.p. 138 - 140°C.

### Beispiel 6: (±)-trans-N-(3-Acetoxy-6-Butoxy-2,2-dimethyl-chroman-4-yl)-N-methylmethansulfonamid

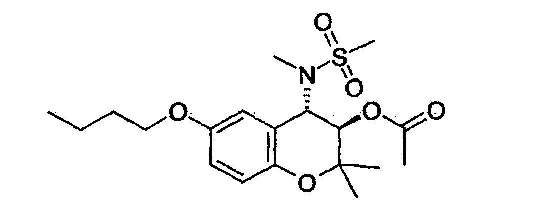

Aus 0,7 g N-(6-Butoxy-3-hydroxy-2,2-dimethyl-chroman-4-yl)-N-methylmethansulfonamid und 6,5 ml Acetanhydrid in 13 ml Pyridin wurden analog Beispiel 2 0,6 g N-(3-Acetoxy-6-Butoxy-2,2-dimethyl-chroman-4-yl)-N-methylmethansulfonamid erhalten; F.p. 87-89°C.

### Beispiel 7: N-(6-Butoxy-2,2-dimethyl-2H-chromen-4-yl)-N-methyl-methansulfonamid

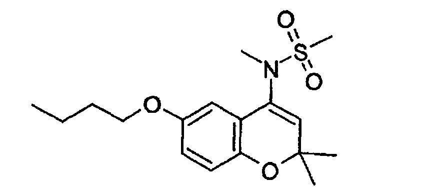

Eine Lösung von 0,5 g (1,3 mmol) N-(6-Butoxy-3-acetoxy-2,2-dimethyl-chroman-4-yl)-N-methyl-methansulfonamid (Beispiel 6) und 1,1 g (7,5 mmol) DBU in 2,5 ml Toluol wurde 60 h auf 105°C erhitzt. Die Reaktionsmischung wurde mit EE verdünnt und mit Salzsäure gewaschen, bis zur sauren Reaktion der wäßrigen Phase. Nach Waschen mit Natriumbicarbonatlösung, Trocknen über Magnesiumsulfat und Einengen i. Vak. erhielt man 0,3 g N-(6-Butoxy-2,2-dimethyl-2H-chromen-4-yl)-N-methyl-methansulfonamid; F.p. 114-116°C.

### Beispiel 8: (±)-trans-N-(6-Propoxy-3-hydroxy-2,2-dimethyl-chroman-4-yl)-N-methylmethansulfonamid

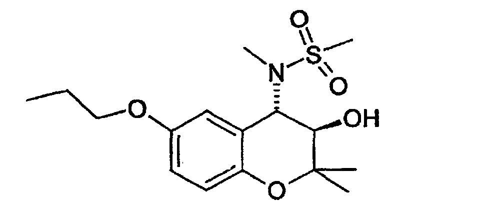

a) N-(3,6-Dihydroxy-2,2-dimethyl-chroman-4-yl)-N-methyl-methansulfonamid 1,0 g (2,6 mmol) (±)-trans-N-(6-Benzyloxy-3-hydroxy-2,2-dimethyl-chroman-4-yl)-N-methyl-methansulfonamid (Beispiel 1) wurden in 100 ml THF/Methanol (1:1) in Gegenwart von Palladium/Kohle bis zum Ende der Wasserstoffaufnahme hydriert. Nach Abfiltrieren des Katalysators und Einengen des Filtrates wurden 0,7 g N-(3,6-Dihydroxy-2,2-dimethyl-chroman-4-yl)-N-methyl-methansulfonamid erhalten; F.p. 204-206°C.
b) Zu einer Lösung von 50 mg (1,6 mmol) 80proz. Natriumhydrid in 2 ml DMF wurden 0,45 g (1,5 mmol) der obigen Verbindung, gelöst in 5 ml DMF, zugetropft. Nach 2 h bei RT wurden 0,27 g (1,6 mmol) 1-lodpropan hinzugefügt und es wurde 2 Tage bei RT gerührt. Nach Aufarbeitung und Umkristallisation aus Isopropanol wurden 0,22 g (±)-trans-N-(6-Propoxy-3-hydroxy-2,2-dimethyl-chroman-4-yl)-N-methyl-methansulfonamid erhalten; F.p. 149-151°C.

### Beispiel 9: N-(6-Propoxy-2,2-dimethyl-2H-chromen-4-yl)-N-methylmethansulfonamid

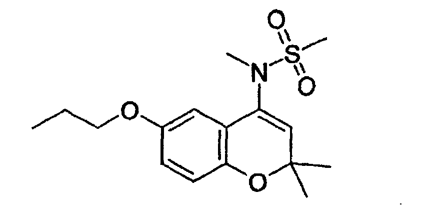

a) Durch Hydrierung von 3,3 g (±)-trans-N-(6-Benzyloxy-3-acetoxy-2,2-dimethylchroman-4-yl)-N-methyl-methansulfonamid (Beispiel 2) in Gegenwart von Pd/C wurden 2,6 g (±)-trans-N-(6-Hydroxy-3-acetoxy-2,2-dimethyl-chroman-4-yl)-N-methyl-methansulfonamid erhalten; F.p. 172-174°C.
b) Anschließende Umsetzung mit DBU in Toluol analog Beispiel 7 lieferte nach Chromatographie an Kieselgel und Umkristallisation aus Toluol / EE 1,2 g N-(6-Hydroxy-2,2-dimethyl-2H-chromen-4-yl)-N-methyl-methansulfonamid; F.p. 130-132°C.
c) 0,5 g N-(6-Hydroxy-2,2-dimethyl-2H-chromen-4-yl)-N-methyl-methansulfonamid wurden analog Beispiel 8b mit 1-lodpropan alkyliert, und man erhielt 0,36 g N-(6-Propoxy-2,2-dimethyl-2H-chromen-4-yl)-N-methyl-methansulfonamid; F.p. 93-95°C.

### Beispiel 10: (±)-trans-N-(6-Benzyloxy-3-hydroxy-2,2-dimethyl-chroman-4-yl)-N-butylmethansulfonamid

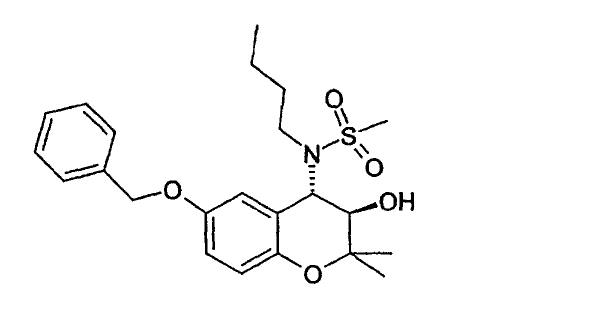

a) (±)-trans-N-(6-Benzyloxy-3-hydroxy-2,2-dimethyl-chroman-4-yl)-methansulfonamid Eine Reaktionsmischung aus 0,47 g (16 mmol) 80proz. Natriumhydrid, 1,75 g (18 mmol) Methansulfonamid und 4,0 g (14 mmol) 6-Benzyloxy-2,2-dimethyl-3,4-epoxychroman (Beispiel 1f) in 25 ml DMSO wurde 36 h auf 50°C erhitzt. Dann wurde die Reaktionsmischung auf Eiswasser gegossen, das ausgefallene Produkt abgesaugt und aus Isopropanol/Petrolether umkristallisiert. Es wurden 2,9 g der Titelverbindung erhalten mit einem Schmelzpunkt von 181-182°C.
b) Eine Lösung von 1,0 g (2,6 mmol) der obigen Verbindung und 90 mg (2,9 mmol) 80proz. Natriumhydrid in 13 ml DMF wurde 1 h bei RT gerührt. Dann wurden 0,51 g (2,8 mmol) 1-lodbutan hinzugefügt und der Ansatz wurde 10 h auf 50°C erhitzt. Die Reaktionsmischung wurde im Vakuum eingeengt, der Rückstand in EE aufgenommen und mit Wasser gewaschen. Nach Reinigung durch Chromatographie an Kieselgel mit Cyclohexan / EE 3:1 wurden 0,5 g (±)-trans-N-(6-Benzyloxy-3-hydroxy-2,2-dimethyl-chroman-4-yl)-N-butyl-methansulfonamid erhalten; F.p. 159-160°C.

### Beispiel 11: N-(6-Benzyloxy-2,2-dimethyl-2H-chromen-4-yl)-N-methylmethansulfonamid

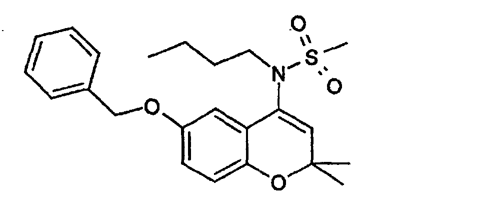

Die Verbindung wurde erhalten aus (±)-trans-N-(6-Benzyloxy-3-hydroxy-2,2-dimethyl-chroman-4-yl)-N-butyl-methansulfonamid (Beispiel 10) analog der in Beispielen 2 und 4 beschriebenen Methode. F.p. 74-76°C.

### Beispiel 12: (±)-trans-[(6-Benzyloxy-3-hydroxy-2,2-dimethyl-chroman-4-yl)-methansulfonyl-amino]-essigsäuremethylester

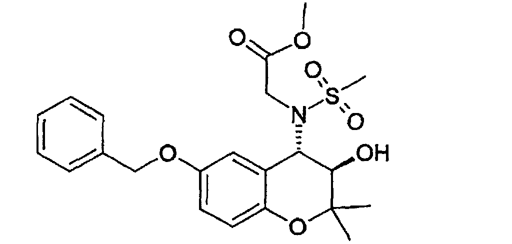

Aus 0,9 g (±)-trans-N-(6-Benzyloxy-3-hydroxy-2,2-dimethyl-chroman-4-yl)-methansulfonamid (Beispiel 10a) wurden durch Alkylierung mit Bromessigsäuremethylester analog Beispiel 10b 0,5 g (±)-trans-[(6-Benzyloxy-3-hydroxy-2,2-dimethyl-chroman-4-yl)-methansulfonyl-amino]-essigsäuremethylester erhalten; F.p. 133-135°C.

### Beispiel 13: (±)-trans-N-[3-Hydroxy-2,2-dimethyl-6-(4,4,4-trifluorbutoxy)-chroman-4-yl]-N-methyl-methansulfonamid

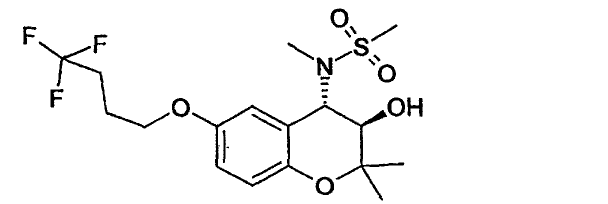

a) (±)-trans-N-[3,6-Dihydroxy-2,2-dimethyl)-chroman-4-yl]-N-methylmethansulfonamid
   2,0 g (5 mmol) (±)-trans-N-(6-Benzyloxy-3-hydroxy-2,2-dimethyl-chroman-4-yl)-N-methyl-methansulfonamid (Beispiel 1) wurden in 200 ml THF/Methanol (1:1) in Gegenwart von Palladium/Aktivkohle (5%Pd) bis zum Ende der Wasserstoffaufnahme bei Normaldruck hydriert. Nach Abfiltrieren des Katalysators und Einengen erhielt man 1,2 g Produkt mit einem Schmelzpunkt von 198-202°C.
b) Zu einer Lösung von 64 mg (2,7 mmol) 80proz. Natriumhydrid in 6 ml DMF wurde eine Lösung von 0,7 g (2,3 mmol) (±)-trans-N-[3,6-Dihydroxy-2,2-dimethyl)-chroman-4-yl]-N-methyl-methansulfonamid in 8 ml DMF zugetropft. Nach einer Stunde wurden 0,56 g (2,4 mmol) 4,4,4-Trifluorbutyliodid zugegeben und es wurde über Nacht bei RT gerührt. Nach Einengen der Reaktionsmischung im Vakuum, Extraktion des Rückstandes mit EE und Wasser und Reinigung durch Chromatographie an Kieselgel wurden 0,71 g (±)-trans-N-[3-Hydroxy-2,2-dimethyl-6-(4,4,4-trifluorbutoxy)-chroman-4-yl]-N-methyl-methansulfonamid erhalten; F.p. 161 - 163°C.

### Beispiel 14: (±)-trans-N-(6-Butoxy-3-hydroxy-2,2-dimethyl-chroman-4-yl)-N-ethylmethansulfonamid

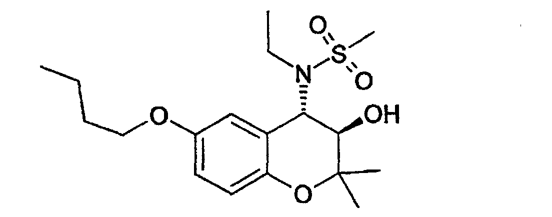

Die Verbindung wurde erhalten aus 6-Butoxy-2,2-dimethyl-3,4-epoxy-chroman (Beispiel 5d) und Methansulfonsäureethylamid analog Beispiel 5 e. Nach Chromatographie an Kieselgel mit Methylenchlorid/Methanol 97 : 3 wurde die Titelverbindung mit einem Schmelzpunkt von 139 - 140°C isoliert.

### Beispiel 15: N-(6-Butoxy-2,2-dimethyl-2H-chromen-4-yl)-N-ethyl-methansulfonamid

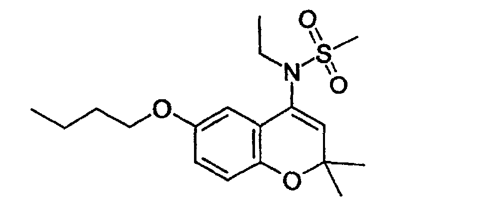

Die Verbindung entstand als Nebenprodukt bei der Herstellung der Verbindung des Beispiels 14 und wurde bei der dort erwähnten Chromatographie als unpolarste Fraktion isoliert. F.p. 68 - 70°C.

### Beispiel 16: (3S, 4R)-(-)-N-(6-Butoxy-3-hydroxy-2,2-dimethyl-chroman-4-yl)-N-methyl-methansulfonamid

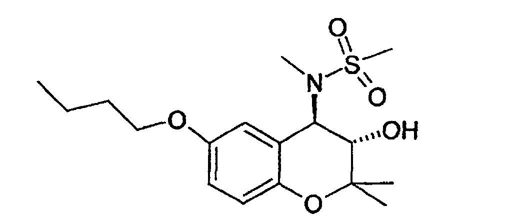

a) (3S, 4S)-(-)-6-Butoxy-2,2-dimethyl-3,4-epoxy-chroman
   Zu einer Lösung von 4,6 g (20 mmol) 6-Butoxy-2,2-dimethyl-2H-chromen (Beispiel 5b) und 0,5 g (0,8 mmol) (S,S)-(+)-N,N'-Bis-(3,5-di-tert.-butylsalicyliden)-1,2-diaminocyclohexan-mangan(III)-chlorid (Jacobsen Katalysator) in 20 ml Methylenchlorid wurden bei 0°C 76 ml (42 mmol) einer 0,55 M Natriumhypochloritlösung, die mit Dinatriumhydrogenphosphat auf pH 11,3 eingestellt war, zugegeben. Die Reaktionsmischung wurde 3 h stark gerührt, dann wurde die organische Phase abgetrennt und die wäßrige noch 2 mal mit Methylenchlorid extrahiert. Nach Chromatographie des Rohproduktes an Kieselgel mit Cyclohexan/EE 9:1 wurden 1,6 g (3S, 4S)-(-)-6-Butoxy-2,2-dimethyl-3,4-epoxychroman erhalten; Drehwert -14,9° (c= 0,6; Methanol).
b) Aus 1 g (3S, 4S)-(-)-6-Butoxy-2,2-dimethyl-3,4-epoxy-chroman wurden analog Beispiel 5 e 0,7 g (3S, 4R)-(-)-N-(6-Butoxy-3-hydroxy-2,2-dimethyl-chroman-4-yl)-N-methyl-methansulfonamid erhalten; F.p. 152-154°C; Drehwert -9,9° (c=0,5; Methanol).

### Beispiel 17: (3S, 4R)-(-)-N-(6-Benzyloxy-3-hydroxy-2,2-dimethyl-chroman-4-yl)-N-methyl-ethansulfonamid

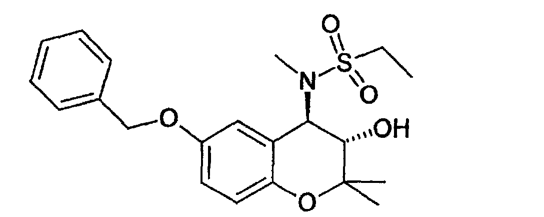

a) (3S, 4S)-6-Benzyloxy-2,2-dimethyl-3,4-epoxy-chroman
   Analog Beispiel 16 a wurden aus 5,0 g 6-Benzyloxy-2,2-dimethyl-2H-chromen (Beispiel 1d) 2,3 g des chiralen Epoxids erhalten.
b) Eine Mischung von 1,0 g (37 mmol) (3S, 4S)-6-Benzyloxy-2,2-dimethyl-3,4-epoxychroman, 1,45 g (74 mmol) N-Methyl-N-trimethylsilyl-ethansulfonamid und 1,17 g Tetrabutylammoniumfluorid-trihydrat (37 mmol) in 5 ml THF wurde 15 h auf 60°C erhitzt. Nach Abdestillieren des Lösungsmittels wurde der Rückstand über eine Kieselgelsäule aufgetrennt und das Produkt aus Isopropanol umkristallisiert. Man erhielt 0,4 g (3S, 4R)-(+)-N-(6-Benzyloxy-3-hydroxy-2,2-dimethyl-chroman-4-yl)-N-methyl-ethansulfonamid; F.p. 172 - 174°C; Drehwert -40,8°.

### Beispiel 18: (3R, 4S)-(+)-N-[-3-Hydroxy-2,2-dimethyl-6-(4,4,4-trifluorbutoxy)-chroman-4-yl]-N-methyl-ethansulfonamid

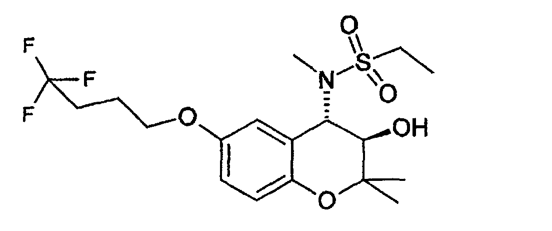

a) (3R, 4R)-6-Benzyloxy-2,2-dimethyl-3,4-epoxy-chroman
   wurde analog Beispiel 18 a erhalten, jedoch unter Verwendung von (R,R)-(+)-N,N'-Bis-(3,5-di-tert.-butylsalicyliden)-1,2-diaminocyclohexan-mangan(III)-chlorid.
b) (3R, 4S)-(+)-N-(6-Benzyloxy-3-hydroxy-2,2-dimethyl-chroman-4-yl)-N-methylethansulfonamid
   Zu einer Lösung von 2,1 g (17 mmol) N-Methyl-ethansulfonamid und 0,2 g (6,7 mmol) 80% Natriumhydrid in 6,5 ml DMSO wurde eine Lösung von 3,7 g (13 mmol) (3R, 4R)-6-Benzyloxy-2,2-dimethyl-3,4-epoxy-chroman in 6,5 ml DMSO zugefügt, und es wurde 20 h auf 60°C erhitzt. Die Reaktionsmischung wurde auf 300 ml Wasser gegeben und das ausgefallene Produkt abgesaugt. Nach Reinigung durch Chromatographie an Kieselgel mit Cyclohexan/EE 8:2 wurden 1,9 g (3R, 4S)-(+)-N-(6-Benzyloxy-3-hydroxy-2,2-dimethyl-chroman-4-yl)-N-methyl-ethansulfonamid erhalten; F.p. 165 - 167°C.
c) (3R, 4S)-(+)-N-(3,6-Dihydroxy-2,2-dimethyl-chroman-4-yl)-N-methylethansulfonamid
   Aus 1,7 g des obigen Benzylethers wurden durch Hydrierung analog Beispiel 13 a 1,3 g der Titelverbindung erhalten.
d) Aus 1,15 g (3R, 4S)-(+)-N-(3,6-Dihydroxy-2,2-dimethyl-chroman-4-yl)-N-methylethansulfonamid wurden durch Alkylierung mit 4,4,4-Trifluorbutyliodid analog Beispiel 13 b 1,1 g (3R, 4S)-(+)-N-[-3-Hydroxy-2,2-dimethyl-6-(4,4,4-trifluorbutoxy)-chroman-4-yl]-N-methyl-ethansulfonamid erhalten; F.p. 173 - 174°C, Drehwert +20,9°.

### Beispiel 19: (3R, 4S)-(+)-N-[-3-Hydroxy-2,2-dimethyl-6-(4,4,4-trifluorbutoxy)-chroman-4-yl]-N-methyl-methansulfonamid

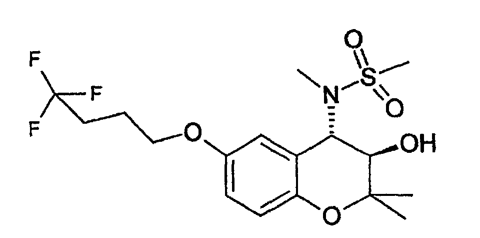

a) 6-(4,4,4-Trifluorbutoxy)-2,2-dimethylchroman-4-on
   Zu einer Lösung von 2,65 g (88 mmol) 80proz. Natriumhydrid in 170 ml DMF wurde eine Lösung von 13.4 g (70 mmol) 2,2-Dimethyl-6-hydroxychroman-4-on (Beispiel 1a) in 250 ml DMF zugetropft. Nach 1 h Rühren bei RT wurden 14,6 g (76 mmol) 4,4,4-Trifluorbutylbromid zugesetzt und über Nacht bei RT stehen gelassen. Die Reaktionsmischung wurde auf 3 l Wasser gegossen und über Nacht stehen gelassen. Das ausgefallene Produkt wurde abgesaugt, mit Wasser gewaschen und im Vakuum getrocknet. Man erhielt 20,0 g 6-(4,4,4-Trifluorbutoxy)-2,2-dimethylchroman-4-on.
b) 6-(4,4,4-Trifluorbutoxy)-2,2-dimethylchroman-4-ol
   Eine Lösung von 20 g (66 mmol) 6-(4,4,4-Trifluorbutoxy)-2,2-dimethylchroman-4-on und 2,5 g (66 mmol) Natriumborhydrid in 100 ml Methanol wurde über Nacht bei RT gerührt. Die Reaktionsmischung wurde auf Eiswasser gegossen, mit Kochsalz versetzt und 4 mal mit EE extrahiert. Nach Trocknen und Einengen der organischen Phase wurden 19,5 g 6-(4,4,4-Trifluorbutoxy)-2,2-dimethylchroman-4-ol erhalten.
c) 6-(4,4,4-Trifluorbutoxy)-2,2-dimethyl-2H-chromen
   Eine Lösung von 19,5 g (64 mmol) 6-(4,4,4-Trifluorbutoxy)-2,2-dimethylchroman-4-ol in 200 ml Toluol wurde mit 0,2 g p-Toluolsulfonsäure-Monohydrat versetzt und 2 ½ h auf 100°C erhitzt. Nach dem Abkühlen wurde der Ansatz 2mal mit je 120 ml Natriumbicarbonatlösung extrahiert und mit Aktivkohle verrührt. Nach Filtration und Einengen im Vakuum wurden 16,7 g 6-(4,4,4-Trifluorbutoxy)-2,2-dimethyl-2H-chromen erhalten.
d) (3R, 4R)-(+)-6-(4,4,4-Trifluorbutoxy)-2,2-dimethyl-3,4-epoxy-chroman
   Zu einer Lösung von 2,86 g (10 mmol) 6-(4,4,4-Trifluorbutoxy)-2,2-dimethyl-2H-chromen und 0,26 g (0,4 mmol) (R,R)-(-)-N,N'-Bis-(3,5-di-tert.butylsalicyliden)-1,2-diaminocyclohexan-mangan(lll)-chlorid (Jacobsen Katalysator) in 11 ml Methylenchlorid wurden bei 0°C 38,5 ml (22 mmol) einer 0,55 M Natriumhypochloritlösung, die mit Dinatriumhydrogenphosphat auf pH 11,3 eingestellt war, zugetropft. Die Reaktionsmischung wurde 1 h stark gerührt, dann wurde die organische Phase abgetrennt und die wäßrige noch einmal mit wenig Methylenchlorid extrahiert. Zur Abtrennung des Katalysators wurde die organische Phase über eine kurze Kieselgelsäule filtriert und die entsprechenden Fraktionen wurden im Vakuum eingeengt. Man erhielt 1,65 g (3R, 4R)-(+)-6-(4,4,4-Trifluorbutoxy)-2,2-dimethyl-3,4-epoxy-chroman als wachsartigen Feststoff; Drehwert ca. +13° (c= 0,5; Methanol).
e) Zu einer Suspension von 0,065 g (2,7 mmol) 80 proz. Natriumhydrid in 3 ml DMSO wurden unter Argon 0,77 g (7,1 mmol) Methansulfonsäuremethylamid zugegeben und es wurde 20 min bei RT gerührt. Dann wurden 1,65 g (5,5 mmol) (3R, 4R)-(+)-6-(4,4,4-Trifluorbutoxy)-2,2-dimethyl-3,4-epoxy-chroman, gelöst in 5 ml DMSO, zugetropft, und der Ansatz wurde 4 Tage bei RT stehen gelassen und dann noch 9 h auf 45 °C erwärmt. Anschließend wurde auf Wasser gegossen, der Niederschlag abgesaugt, im Vakuum gut getrocknet, und man erhielt 1,9 g (3R, 4S)-(+)-N-[-3-Hydroxy-2,2-dimethyl-6-(4,4,4-trifluorbutoxy)-chroman-4-yl]-N-methylmethansulfonamid, das aus wenig Isopropanol umkristallisiert wurde (-> 1,4 g; F.p. 178 - 179°C; optische Reinheit (chirale HPLC) 100%).

### Beispiel 20: (3S, 4R)-(-)-N-[-3-Hydroxy-2,2-dimethyl-6-(4,4,4-trifluorbutoxy)-chroman-4-yl]-N-methyl-methansulfonamid

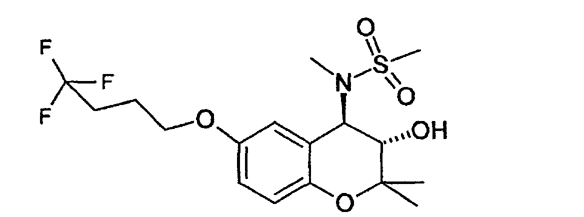

Die Verbindung wurde erhalten analog Beispiel 20, jedoch unter Verwendung von (S,S)-(-)-N,N'-Bis-(3,5-di-tert.-butylsalicyliden)-1,2-diaminocyclohexanmangan(III)-chlorid als Epoxidierungskatalysator. F.p. 179°C; optische Reinheit (chirale HPLC) 100%.

### Beispiel 21: (±)-trans-N-[3-Acetoxy-6-(4,4,4-trifluorbutoxy)-2,2-dimethylchroman-4-yl]-N-methyl-methansulfonamid

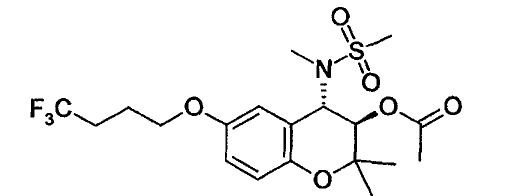

Aus 3,5 g (±)-trans-N-[3-Hydroxy-2,2-dimethyl-6-(4,4,4-trifluorbutoxy)-chroman-4-yl]-N-methyl-methansulfonamid (Beispiel 13) and 28 ml Acetanhydrid in 55 ml Pyridin wurden analog Beispiel 2 3,7 g (±)-trans-N-[3-Acetoxy-6-(4,4,4-trifluorbutoxy)-2,2-dimethylchroman-4-yl]-N-methyl-methanesulfonamid erhalten; F.p. 106°C.

### Beispiel 22: N-[6-(4,4,4-Trifluorbutoxy)-2,2-dimethyl-2H-chromen-4-yl]-N-methylmethansulfonamid

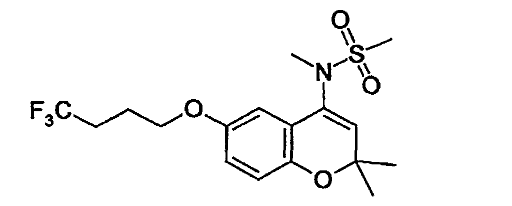

Eine Lösung von 3,5 g (±)-trans-N-[3-Acetoxy-6-(4,4,4-trifluorbutoxy)-2,2-dimethylchroman-4-yl]-N-methyl-methansulfonamid (Beispiel 21) and 6,8 g DBU in 30 ml Toluol wurde 20 h auf 100°C erhitzt. Die Reaktionsmischung wurde mit EE verdünnt und mit Salzsäure gewaschen, bis zur sauren Reaktion der wäßrigen Phase. Nach Waschen mit Natriumbicarbonatlösung, Trocknen über Magnesiumsulfat, Einengen i. Vak. und Verrühren mit Heptan erhielt man 1,7 g N-[6-(4,4,4-Trifluorbutoxy)-2,2-dimethyl-2H-chromen-4-yl]-N-methyl-methansulfonamid; F.p. 118°C.

### Beispiel 23: (3R,4S)-{[3-Hydroxy-2,2-dimethyl-6-(4,4,4-trifluor-butoxy)-chroman-4-yl]-methansulfonyl-amino}-essigsäuremethylester

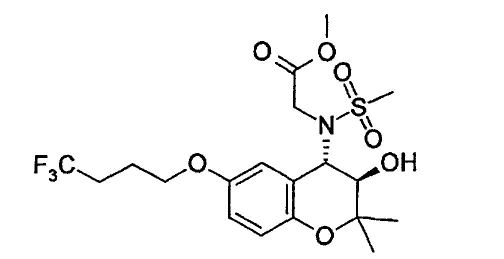

a) (3R,4S)- N-[3-Hydroxy-2,2-dimethyl-6-(4,4,4-trifluor-butoxy)-chroman-4-yl]-methansulfonamid
   0,82 g (8,6 mmol) Methansulfonamid wurden unter Argon zu einer Suspension von 0,2 g (6,6 mmol) 80proz. Natriumhydrid in 3,5 ml DMSO hinzugefügt und die Mischung wurde 30 min bei RT gerührt. Dann wurden 2,0 g (6,6 mmol) (3R,4R)-(+)-6-(4,4,4-Trifluorbutoxy)-2,2-dimethyl-3,4-epoxychroman (Beispiel 19d), gelöst in 6 ml DMSO, hinzugetropft and der Ansatz wurde 20 h auf 60°C erhitzt. Anschließend wurde die Mischung auf Wasser gegossen, der ausgefallene Niederschlag abgesaugt und im Vakuum getrocknet, und man erhielt 1,6 g (3R,4S)- N-[3-Hydroxy-2,2-dimethyl-6-(4,4,4-trifluor-butoxy)-chroman-4-yl]-methansulfonamid; F.p. 186°C.
b) Eine Lösung von 0,5 g (1,3 mmol) der obigen Verbindung und 0,05 g (1,7 mmol) 80proz. Natriumhydrid in 5 ml DMF wurde 1 h bei RT gerührt. Dann wurden 0,2 g (1,3 mmol) Bromessigsäuremethylester hinzugefügt, und der Ansatz wurde über Nacht bei RT gerührt. Nach Aufarbeitung und Reinigung durch Chromatographie über Kieselgel wurden 0,2 g (3R,4S)-{[3-Hydroxy-2,2-dimethyl-6-(4,4,4-trifluorbutoxy)-chroman-4-yl]-methansulfonyl-amino}-essigsäuremethylester erhalten.

### Beispiel 24:

Analog den zuvor beschriebenen Methoden können die folgenden Verbindungen hergestellt werden, die von besonderer Bedeutung sind:
a) trans-N-[3-Hydroxy-2,2-dimethyl-6-(4,4,4-trifluorbutoxy)chroman-4-yl]-N-ethyl-methansulfonamid;
b) trans-N-[3-Hydroxy-2,2-dimethyl-6-(4,4,4-trifluorbutoxy)chroman-4-yl]-N-(2,2,2-trifluorethyl)-methansulfonamid;
c) trans-N-[3-Hydroxy-2,2-dimethyl-6-(4,4,4-trifluorbutoxy)chroman-4-yl]-N-propyl-methansulfonamid;
d) trans-N-[3-Hydroxy-2,2-dimethyl-6-(4,4,4-trifluorbutoxy)chroman-4-yl]-N-ethyl-ethansulfonamid;
e) trans-N-[3-Hydroxy-2,2-dimethyl-6-(4,4,4-trifluorbutoxy)chroman-4-yl]-N-(2,2,2-trifluorethyl)-ethansulfonamid;
f) trans-N-[3-Hydroxy-2,2-dimethyl-6-(4,4,4-trifluorbutoxy)chroman-4-yl]-N-propyl-ethansulfonamid;
g) trans-N-[3-Hydroxy-2,2-dimethyl-6-(3,3,3-trifluorpropoxy)chroman-4-yl]-N-methyl-methansulfonamid;
h) trans-N-[3-Hydroxy-2,2-dimethyl-6-(3,3,3-trifluorpropoxy)chroman-4-yl]-N-ethyl-methansulfonamid;
i) trans-N-[3-Hydroxy-2,2-dimethyl-6-(cyclopropylmethoxy)chroman-4-yl]-N-methyl-methansulfonamid;
j) trans-N-[3-Hydroxy-2,2-dimethyl-6-(cyclopropylmethoxy)chroman-4-yl]-N-methyl-ethansulfonamid.

### Pharmakologische Untersuchungen

I_{sK}-Kanäle aus Mensch, Ratte oder Meerschweinchen wurden in Xenopus-Oozyten exprimiert. Hierfür wurden zuerst Oozyten aus Xenopus Laevis isoliert und defollikuliert. Anschließend wurde in diese Oozyten in vitro synthetisierte I_{sK}-kodierende RNA injiziert. Nach 2 - 8 Tagen I_{sK}-Proteinexpression wurden an den Oozyten mit der Zwei-Mikroelektroden Voltage-Clamp Technik I_{sK}-Ströme gemessen. Die I_{sK}-Kanäle wurden hierbei in der Regel mit 15 s dauernden Spannungssprüngen auf -10 mV aktiviert. Das Bad wurde mit einer Lösung der nachfolgenden Zusammensetzung durchspült: NaCl 96 mM, KCI 2 mM, CaCl₂ 1,8 mM, MgCl₂ 1 mM, HEPES 5 mM (titriert mit NaOH auf pH 7,5). Diese Experimente wurden bei Raumtemperatur durchgeführt. Zur Datenerhebung und Analyse wurden eingesetzt: Geneclamp Verstärker (Axon Instruments, Foster City, USA) und MacLab D/A-Umwandler und Software (ADInstruments, Castle Hill, Australia). Die erfindungsgemäßen Substanzen wurden getestet, indem sie in unterschiedlichen Konzentrationen der Badlösung zugefügt wurden. Die Effekte der Substanzen wurden als prozentuale Inhibition des I_{sK}-Kontrollstromes berechnet, der erhalten wurde, wenn der Lösung keine Substanz zugesetzt wurde. Die Daten wurden anschließend mit der Hill-Gleichung extrapoliert, um die Hemmkonzentrationen IC₅₀ für die jeweiligen Substanzen zu bestimmen.

### Literatur:

A.E. Busch, H.-G. Kopp, S. Waldegger, I. Samarzija, H. Süßbrich, G. Raber, K. Kunzelmann, J. P. Ruppersberg und F. Lang; "Inhibition of both exogenously expressed I_{sK} and endogenous K⁺ channels in Xenopus oocytes by isosorbiddinitrate"; J. Physiol. 491 (1995), 735-741; T. Takumi, H. Ohkubo und S. Nakanishi; "Cloning of a membrane protein that induces a slow voltage-gated potassium current"; Science 242 (1989), 1042-1045;
M.D. Varnum, A.E. Busch, C.T. Bond, J. Maylie und J.P. Adelman; "The minK channel underlies the cardiac potassium current and mediates species-specific responses to protein kinase"; C. Proc. Natl. Acad. Sci. USA 90 (1993), 11528-11532.

Auf die beschriebene Weise unter Verwendung des humanen I_{sK}-Proteins wurden für die erfindungsgemäßen Verbindungen folgende IC₅₀-Werte bestimmt:

| Verbindung | IC-50 [µM] |
|---|---|
| Beispiel 1 | 0,2 |
| Beispiel 2 | 13 |
| Beispiel 3 | 6,5 |
| Beispiel 4 | 1,6 |
| Beispiel 5 | 0,25 |
| Beispiel 6 | >10 |
| Beispiel 7 | 1,6 |
| Beispiel 8 | 0,9 |
| Beispiel 9 | 2,6 |
| Beispiel 10 | 0,4 |
| Beispiel 11 | 1,6 |
| Beispiel 12 | 0,8 |
| Beispiel 13 | 0,2 |
| Beispiel 14 | 0,2 |
| Beispiel 15 | 1,3 |
| Beispiel 16 | 0,4 |
| Beispiel 17 | 0,4 |
| Beispiel 18 | 0,1 |
| Beispiel 19 | 0,1 |
| Beispiel 20 | 0,4 |
| Beispiel 21 | ∼ 10 |
| Beispiel 22 | 0,6 |
| Beispiel 23a | 1,7 |

## Patentansprüche

1. Verbindungen der Formel I, 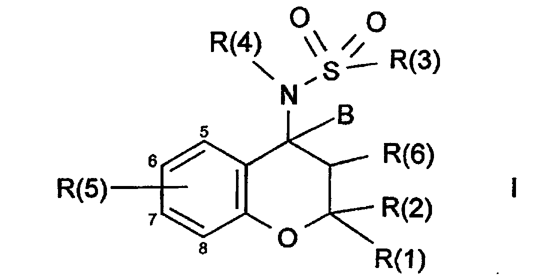 worin R(5) in einer der mit 5, 6, 7 und 8 **gekennzeichneten** Positionen gebunden ist und worin bedeuten:
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(1) und R(2)
gemeinsam eine Alkylenkette mit 2, 3, 4, 5, 6, 7, 8, 9 oder 10 C-Atomen;
R(3) R(10)-CₙH₂ₙ-NR(11)- oder R(10)-CₙH₂ₙ-,
wobei eine CH₂-Gruppe in den Gruppen CₙH₂ₙ ersetzt sein kann durch -O-, -CO-, -S-, -SO-, -SO₂- oder -NR(12a)-; R(12a) Wasserstoff, Methyl oder Ethyl;
R(10) Wasserstoff, Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen;
oder
R(10) und R(11)
gemeinsam eine Bindung, sofern n nicht kleiner als 3 ist;
oder
R(3) gemeinsam mit R(4)
eine Alkylenkette mit 3, 4, 5, 6, 7 oder 8 C-Atomen,
wobei eine CH₂-Gruppe der Alkylenkette durch -O-, -CO-, -S-, -SO-, -SO₂- oder -NR(12a)- ersetzt sein kann;
R(12a) Wasserstoff, Methyl oder Ethyl;
R(4) R(13)-CᵣH₂ᵣ,
wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂- oder -NR(14)-;
R(14) Wasserstoff oder Alkyl mit 1, 2 oder 3 C-Atomen;
R(13) CH₃, CF₃, C₂F₅, C₃F₇, Cycloalkyl mit 3, 4, 5, 6, 7 oder 8 C-Atomen, NR(15)R(16), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
r Null, 1, 2, 3, 4, 5, 6, 7, 8, 9, 10, 11, 12, 13, 14, 15, 16, 17, 18, 19 oder 20;
R(5) -Y-CₛH₂ₛ-R(18) oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -S- oder-NR(10c)-;
R(10c) Wasserstoff oder Alkyl mit 1, 2 oder 3 C-Atomen;
s 1, 2, 3, 4, 5, 6, 7 oder 8;
R(18) Wasserstoff, CF₃, C₂F₅, C₃F₇, Cycloalkyl mit 3, 4, 5, 6, 7 oder 8 C-Atomen, -COOR(21), NR(15a)R(16a), ein unsubstituierter N-haltiger Heterocyclus mit 1, 2, 3, 4, 5, 6, 7, 8 oder 9 C-Atomen, Phenyl oder Thienyl,
wobei Phenyl und Thienyl unsubstituiert sind oder substituiert mit 1 oder 2 Substituenten, ausgewählt aus der Gruppe bestehend aus F, Cl, Br, I, CF₃, Methyl, Methoxy, Sulfamoyl, Methylsulfonyl und Methylsulfonylamino;
R(15a) und R(16a)
gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
R(21) Wasserstoff oder Alkyl mit 1, 2 oder 3 C-Atomen;
R(6) OR(10d) oder OCOR(10d);
R(10d) Wasserstoff oder Alkyl mit 1, 2 oder 3 C-Atomen;
B Wasserstoff;
oder
R(6) und B
gemeinsam eine Bindung; sowie ihre physiologisch verträglichen Salze.

2. Verbindungen der Formel I nach Anspruch I, in der bedeuten:
R(1) und R(2)
unabhängig voneinander Wasserstoff, CF₃, C₂F₅, C₃F₇ oder Alkyl mit 1, 2, 3, 4, 5 oder 6 C-Atomen;
oder
R(1) und R(2)
gemeinsam eine Alkylenkette mit 2, 3, 4, 5, 6, 7, 8, 9 oder 10 C-Atomen;
R(3) R(10)-CₙH₂ₙ;
R(10) Methyl, CF₃, C₂F₅, oder C₃F₇;
n Null, 1, 2, 3, 4, 5 oder 6;
R(4) R(13)-CᵣH₂ᵣ,
wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂- oder -NR(14)-;
R(14) Wasserstoff oder Alkyl mit 1, 2 oder 3 C-Atomen;
R(13) CH₃, CF₃, C₂F₅, C₃F₇, Cycloalkyl mit 3, 4, 5, 6, 7 oder 8 C-Atomen, NR(15)R(16), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
r Null, 1, 2, 3, 4, 5, 6, 7, 8, 9, 10, 11 oder 12;
R(5) -Y-CₛH₂ₛ-R(18) oder Phenyl,
das unsubstituiert ist oder substituiert mit 1 oder 2 Substituenten, ausgewählt aus der Gruppe bestehend aus F, Cl, Br, 1, CF₃, NO₂, CN, NH₂, OH, Methyl, Ethyl, Methoxy, Dimethylamino, Sulfamoyl, Methylsulfonyl und Methylsulfonylamino;
Y -O- oder -S-;
s 1, 2, 3, 4, 5, 6, 7 oder 8;
R(18) Wasserstoff, CF₃, C₂F₅, C₃F₇, Cycloalkyl mit 3, 4, 5, 6, 7 oder 8 C-Atomen, -COOR(21), NR(15a)R(16a), ein unsubstituierter N-haltiger Heterocyclus mit 1, 2, 3, 4, 5, 6, 7, 8 oder 9 C-Atomen, Phenyl oder Thienyl,
wobei Phenyl und Thienyl unsubstituiert sind oder substituiert mit 1 oder 2 Substituenten, ausgewählt aus der Gruppe bestehend aus F, Cl, Br, I, CF₃, Methyl, Methoxy, Sulfamoyl, Methylsulfonyl und Methylsulfonylamino;
R(15a) und R(16a)
gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
R(21) Alkyl mit 1, 2 oder 3 C-Atomen;
R(6) OR(10d) oder OCOR(10d);
R(10d) Wasserstoff oder Alkyl mit 1, 2 oder 3 C-Atomen;
B Wasserstoff;
oder
R(6) und B
gemeinsam eine Bindung; sowie ihre physiologisch verträglichen Salze.

3. Verbindungen der Formel I nach Ansprüchen 1 oder 2, in der R(5) an der mit 6 **gekennzeichneten** Position gebunden ist, also Verbindungen der Formel la, 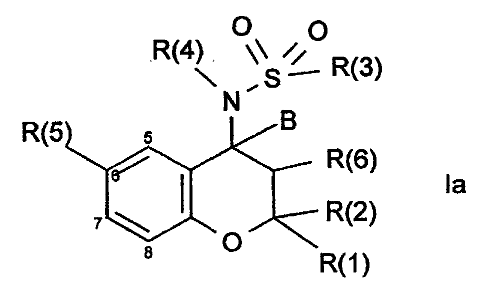 worin die Reste R(1), R(2), R(3), R(4), R(5), R(6) und B die in Anspruch 2 angegebenen Bedeutungen haben; sowie ihre physiologisch verträglichen Salze.

4. Verbindungen der Formel I a nach einem oder mehreren der Ansprüche 1 bis 3, in der bedeuten:
R(1) und R(2)
unabhängig voneinander Wasserstoff, CF₃ oder Alkyl mit 1, 2 oder 3 C-Atomen;
oder
R(1) und R(2)
gemeinsam eine Alkylenkette mit 2, 3, 4, 5 oder 6 C-Atomen;
R(3) R(10)-CₙH₂ₙ;
R(10) Methyl oder CF₃;
n Null, 1 oder 2;
R(4) R(13)-CᵣH₂ᵣ,
wobei eine CH₂-Gruppe der Gruppe CᵣH₂ᵣ ersetzt sein kann durch -O-, -CO-, -CO-O-, -O-CO-, -S-, -SO-, -SO₂- oder -NR(14)-; R(14) Wasserstoff oder Alkyl mit 1, 2 oder 3 C-Atomen;
R(13) CH₃, CF₃, NR(15)R(16), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
r Null, 1, 2, 3, 4, 5, 6, 7 oder 8;
R(5) -Y-CₛH₂ₛ-R(18);
Y -O-;
s 1, 2, 3, 4, 5 oder 6;
R(18) Wasserstoff, CF₃, -COOR(21), NR(15a)R(16a), ein unsubstituierter N-haltiger Heterocyclus mit 1, 2, 3, 4, 5, 6, 7, 8 oder 9 C-Atomen, Phenyl oder Thienyl,
wobei Phenyl und Thienyl unsubstituiert sind oder substituiert mit 1 oder 2 Substituenten, ausgewählt aus der Gruppe bestehend aus F, Cl, Br, I, CF₃, Methyl, Methoxy, Sulfamoyl, Methylsulfonyl und Methylsulfonylamino;
R(15a) und R(16a)
gemeinsam eine Kette von 4 oder 5 Methylengruppen, von denen eine CH₂-Gruppe durch -O-, -S-, -NH-, -N(Methyl)- oder -N(Benzyl)- ersetzt sein kann;
R(21) Alkyl mit 1, 2 oder 3 C-Atomen;
R(6) OH;
B Wasserstoff;
oder R(6) und B
gemeinsam eine Bindung; sowie ihre physiologisch verträglichen Salze.

5. Verbindungen der Formel I a nach einem oder mehreren der Ansprüche 1 bis 4, in der bedeuten:
R(1) und R(2)
Methyl;
R(3) Methyl oder Ethyl;
R(4) R(13)-CᵣH₂ᵣ,
wobei eine CH₂-Gruppe der Gruppe CᵣH₂ᵣ ersetzt sein kann durch -O-, -CO-, -CO-O- oder -O-CO; R(13) CH₃ oder CF₃;
r Null, 1, 2, 3, 4, 5, 6, 7 oder 8;
R(5)
-Y-CₛH₂ₛ-R(18);
Y -O-;
s 1, 2, 3, 4, 5 oder 6;
R(18) Wasserstoff, CF₃, -COOR(21), Phenyl oder Thienyl,
wobei Phenyl und Thienyl unsubstituiert sind oder substituiert mit 1 oder 2 Substituenten, ausgewählt aus der Gruppe bestehend aus F, Cl, Br, CF₃, Methyl oder Methoxy;
R(21) Alkyl mit 1, 2 oder 3 C-Atomen;
R(6) und B
gemeinsam eine Bindung; sowie ihre physiologisch verträglichen Salze.

6. Verbindungen der Formel la nach einem oder mehreren der Ansprüche 1 bis 4, in der bedeuten:
R(1) und R(2)
Methyl;
R(3) Methyl oder Ethyl,
R(4) R(13)-CᵣH₂ᵣ,
wobei eine CH₂-Gruppe der Gruppe CᵣH₂ᵣ ersetzt sein kann durch -O-, -CO-, -CO-O- oder -O-CO; R(13) CH₃ oder CF₃;
r Null, 1, 2, 3, 4, 5, 6, 7 oder 8;
R(5) -Y-CₛH₂ₛ-R(18);
Y -O-;
s 1, 2, 3, 4, 5 oder 6;
R(18) Wasserstoff, CF₃, Phenyl oder Thienyl, wobei Phenyl und Thienyl unsubstituiert sind oder substituiert mit 1 oder 2 Substituenten, ausgewählt aus der Gruppe bestehend aus F, Cl, Br, CF₃, Methyl oder Methoxy;
R(6) OH;
B Wasserstoff; sowie ihre physiologisch verträglichen Salze.

7. Verfahren zur Herstellung von Verbindungen der Formel I gemäß einem oder mehreren der Ansprüche 1 bis 6, **dadurch gekennzeichnet, daß** man
a) eine Verbindung der Formel II, 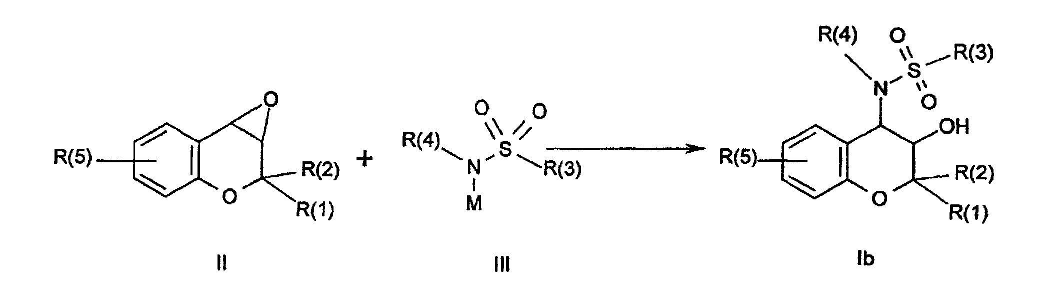 worin R(1), R(2) und R(5) die in den Ansprüchen 1 bis 6 angegebenen Bedeutungen besitzen, mit einem Sulfonamid der Formel III, worin R(3) und R(4) die in den Ansprüchen 1 bis 6 angegebenen Bedeutungen haben und M für Wasserstoff oder ein Metalläquivalent oder einen Trialkylsilylrest steht, umsetzt zu einem Chromanol der Formel Ib;
oder daß man
b) eine Verbindung der Formel Ib mit einem Alkylierungsmittel der Formel R(10d)-L oder einem Acylierungsmittel der Formel R(10d)-COL bzw. einem Anhydrid der Formel (R(10d)-CO)₂O, worin R(10d) die in den Ansprüchen 1 bis 4 angegeben Bedeutungen besitzt und L eine nucleofuge Fluchtgruppe bedeutet, umsetzt in an sich bekannter Weise im Sinne einer Alkylierungs- bzw. Acylierungsreaktion zu einer Verbindung der Formel Ic, worin R(6) OR(10d) oder OCOR(10d) bedeutet; 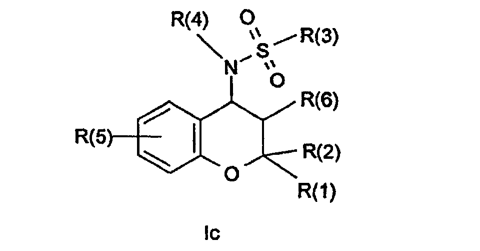 oder daß man
c) eine Verbindung der Formel lc, 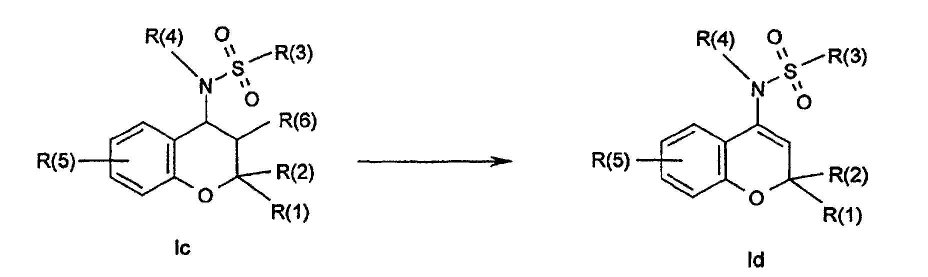
worin R(1), R(2), R(3), R(4) und R(5) die in den Ansprüchen 1 bis 6 angegebenen Bedeutungen haben und R(6) OCOCH₃ bedeutet, im Sinne einer Eliminierungsreaktion zu einer Verbindung der Formel Id, worin R(1), R(2), R(3), R(4) und R(5) die in den Ansprüchen 1 bis 6 angegebenen Bedeutungen haben, umwandelt.

8. Verbindungen der Formel I nach einem oder mehreren der Ansprüche 1 bis 6 und ihre physiologisch verträglichen Salze zur Anwendung als Arzneimittel.

9. Pharmazeutische Zubereitung, enthaltend eine wirksame Menge mindestens einer Verbindung der Formel I nach einem oder mehreren der Ansprüche 1 bis 6 und/oder eines physiologisch verträglichen Salzes davon als Wirkstoff, zusammen mit pharmazeutisch annehmbaren Träger- und Zusatzstoffen und gegebenenfalls noch einem oder mehreren anderen pharmakologischen Wirkstoffen.

10. Verwendung einer Verbindung der Formel I nach einem oder mehreren der Ansprüche 1 bis 6 und/oder eines physiologisch verträglichen Salzes davon zur Herstellung eines Medikaments mit K⁺-Kanal-blockierender Wirkung zur Therapie und Prophylaxe von K⁺-Kanal mediierten Krankheiten.

11. Verwendung einer Verbindung der Formel I nach einem oder mehreren der Ansprüche 1 bis 6 und/oder eines physiologisch verträglichen Salzes davon zur Herstellung eines Medikaments zum Inhibieren der Magensäuresekretion.

12. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von Ulcera des Magens oder des intestinalen Bereiches.

13. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe der Refluxösophagitis.

14. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von Durchfallerkrankungen.

15. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe aller Typen von Arrhythmien, einschließlich atrialer, ventrikulärer und supraventrikulärer Arrhythmien.

16. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von Herzrhythmusstörungen, die durch Aktionspotential-Verlängerung behoben werden können.

17. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von atrialer Fibrillation oder atrialem Flattern.

18. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von Reentry-Arrhythmien oder zur Verhinderung des plötzlichen Herztodes infolge von Kammerflimmern.

19. Verwendung einer Verbindung der Formel I nach einem oder mehreren der Ansprüche 1 bis 6 und/oder eines physiologisch verträglichen Salzes davon zur Herstellung eines Medikaments zur Therapie der Herzinsuffizienz, insbesondere der Stauungsherzinsuffizienz (Congestive Heart Failure).

20. Verwendung einer Verbindung der Formel I nach einem oder mehreren der Ansprüche 1 bis 6 und/oder eines physiologisch verträglichen Salzes davon zum Herstellen eines Medikaments zum Inhibieren der stimulierten Magensäuresekretion, zur Therapie oder Prophylaxe von Ulcera des Magens oder des intestinalen Bereiches, der Refluxösophagitis, von Durchfallerkrankungen, zur Therapie oder Prophylaxe von Arrhythmien, einschließlich atrialer, ventrikulärer und supraventrikulärer Arrhythmien, atrialer Fibrillation und atrialem Flattern und von Reentry-Arrhythmien, oder zur Verhinderung des plötzlichen Herztodes infolge von Kammerflimmern.

21. Pharmazeutische Zubereitung, enthaltend eine wirksame Menge mindestens einer Verbindung der Formel I nach einem oder mehreren der Ansprüche 1 bis 6 und/oder eines physiologisch verträglichen Salzes davon sowie eines Beta-blockers als Wirkstoffe, zusammen mit pharmazeutisch annehmbaren Träger- und Zusatzstoffen.

## Claims

1. A compound of the formula I, 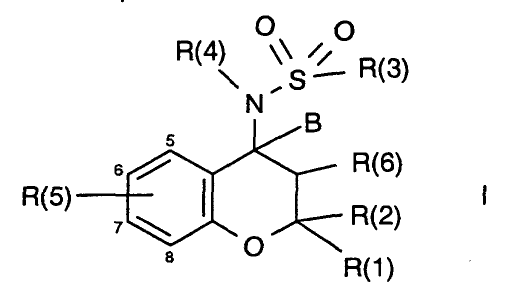 in which R(5) is attached to one of the positions labeled 5, 6, 7 and 8 and in which:
R(1) and R(2)
independently of one another are hydrogen, CF₃, C₂F₅, C₃F₇, alkyl having 1, 2, 3, 4, 5 or 6 carbon atoms or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino; or
R(1) and R(2)
together are an alkylene chain having 2, 3, 4, 5, 6, 7, 8, 9 or 10 carbon atoms;
R(3) is R(10)-CₙH₂ₙ-NR(11)- or R(10)-CₙH₂ₙ-,
where one CH₂ group in the groups CₙH₂ₙ may be replaced by -O-, -CO-, -S-, -SO-, -SO₂- or -NR(12a)-; R(12a) is hydrogen, methyl or ethyl;
R(10) is hydrogen, methyl, cycloalkyl having 3, 4, 5, 6, 7 or 8 carbon atoms, CF₃, C₂F₅ or C₃F₇;
n is zero, 1, 2, 3, 4, 5, 6, 7, 8, 9 or 10;
R(11) is hydrogen or alkyl having 1, 2, 3, 4, 5 or 6 carbon atoms;
or
R(10) and R(11)
together are a bond, if n is not less than 3;
or
R(3) together with R(4)
is an alkylene chain having 3, 4, 5, 6, 7 or 8 carbon atoms,
where a CH₂ group of the alkylene chain may be replaced by -O-, -CO-, -S-, -SO-, -SO₂- or -NR(12a)-; R(12a) is hydrogen, methyl or ethyl;
R(4) is R(13)-CᵣH₂ᵣ,
where a CH₂ group of the group CᵣH₂ᵣ may be replaced by -O-, -CH=CH-, -C≡C-, -CO-, -CO-O-, -O-CO-, -S-, -SO-, -SO₂- or -NR(14)-;
R(14) is hydrogen or alkyl having 1, 2 or 3 carbon atoms;
R(13) is CH₃, CF₃, C₂F₅, C₃F₇, cycloalkyl having 3, 4, 5, 6, 7 or 8 carbon atoms, NR(15)R(16), phenyl or a nitrogen-containing heterocycle having 1, 2, 3, 4, 5, 6, 7, 8 or 9 carbon atoms,
where phenyl and the nitrogen-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15) and R(16)
together are a chain of 4 or 5 methylene groups of which one CH₂ group may be replaced by -O-, -S-, -NH-, -N(methyl)- or -N(benzyl)-;
r is zero, 1, 2, 3, 4, 5, 6, 7, 8, 9, 10, 11, 12, 13, 14, 15, 16, 17, 18, 19 or 20;
R(5) is -Y-CₛH₂ₛ-R(18) or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
Y is -O-, -S- or-NR(10c)-;
R(10c) is hydrogen or alkyl having 1, 2 or 3 carbon atoms;
s is 1, 2, 3, 4, 5, 6, 7 or 8;
R(18) is hydrogen, CF₃, C₂F₅, C₃F₇, cycloalkyl having 3, 4, 5, 6, 7 or 8 carbon atoms, -COOR(21), NR(15a)R(16a), an unsubstituted nitrogen-containing heterocycle having 1, 2, 3, 4, 5, 6, 7, 8 or 9 carbon atoms, phenyl or thienyl,
where phenyl and thienyl are unsubstituted or substituted by 1 or 2 substituents selected from the group consisting of F, Cl, Br, I, CF₃, methyl, methoxy, sulfamoyl, methylsulfonyl and methylsulfonylamino;
R(15a) and R(16a)
together are a chain of 4 or 5 methylene groups of which one CH₂ group may be replaced by -O-, -S-, -NH-, -N(methyl)- or -N(benzyl)-;
R(21) is hydrogen or alkyl having 1, 2 or 3 carbon atoms;
R(6) is OR(10d) or OCOR(10d);
R(10d) is hydrogen or alkyl having 1, 2 or 3 carbon atoms;
B is hydrogen;
or
R(6) and B
together are a bond; and their physiologically tolerable salts.

2. The compound of the formula I as claimed in claim 1 in which:
R(1) and R(2)
independently of one another are hydrogen, CF₃, C₂F₅, C₃F₇ or alkyl having 1, 2, 3, 4, 5 or 6 carbon atoms; or
R(1) and R(2)
together are an alkylene chain having 2, 3, 4, 5, 6, 7, 8, 9 or 10 carbon atoms;
R(3) is R(10)-CₙH₂ₙ;
R(10) is methyl, CF₃, C₂F₅ or C₃F₇;
n is zero, 1, 2, 3, 4, 5 or 6;
R(4) is R(13)-CᵣH₂ᵣ,
where one CH₂ group of the group CᵣH₂ᵣ may be replaced by -O-, -CH=CH-, -C≡C-, -CO-, -CO-O-, -O-CO-, -S-, -SO-, -SO₂- or -NR(14)-; R(14) is hydrogen or alkyl having 1, 2 or 3 carbon atoms;
R(13) is CH₃, CF₃, C₂F₅, C₃F₇, cycloalkyl having 3, 4, 5, 6, 7 or 8 carbon atoms, NR(15)R(16), phenyl or a nitrogen-containing heterocycle having 1, 2, 3, 4, 5, 6, 7, 8 or 9 carbon atoms,
where phenyl and the nitrogen-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15) and R(16)
together are a chain of 4 or 5 methylene groups of which one CH₂ group may be replaced by -O-, -S-, -NH-, -N(methyl)- or -N(benzyl)-;
r is zero, 1, 2, 3, 4, 5, 6, 7, 8, 9, 10, 11 or 12;
R(5) is -Y-CₛH₂ₛ-R(18) or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
Y is -O- or -S-;
s is 1, 2, 3, 4, 5, 6, 7 or 8;
R(18) is hydrogen, CF₃, C₂F₅, C₃F₇, cycloalkyl having 3, 4, 5, 6, 7 or 8 carbon atoms, -COOR(21), NR(15a)R(16a), an unsubstituted nitrogen-containing heterocycle having 1, 2, 3, 4, 5, 6, 7, 8 or 9 carbon atoms, phenyl or thienyl,
where phenyl and thienyl are unsubstituted or substituted by 1 or 2 substituents selected from the group consisting of F, Cl, Br, I, CF₃, methyl, methoxy, sulfamoyl, methylsulfonyl and methylsulfonylamino;
R(15a) and R(16a)
together are a chain of 4 or 5 methylene groups of which one CH₂ group may be replaced by -O-, -S-, -NH-, -N(methyl)- or -N(benzyl)-;
R(21) is alkyl having 1, 2 or 3 carbon atoms;
R(6) is OR(10d) or OCOR(10d);
R(10d) is hydrogen or alkyl having 1, 2 or 3 carbon atoms;
B is hydrogen;
or
R(6) and B
together are a bond; and their physiologically tolerable salts.

3. The compound of the formula I as claimed in claims 1 or 2, in which R(5) is attached to the position labeled 6, i.e. to compounds of the formula la, 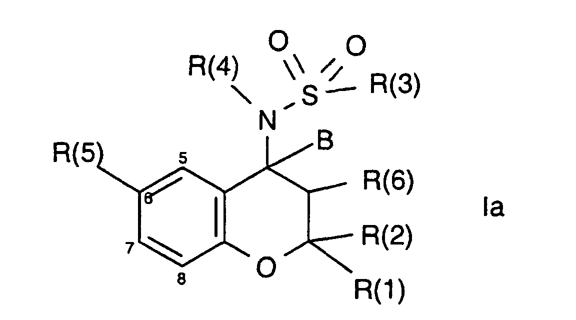 in which the radicals R(1), R(2), R(3), R(4), R(5), R(6) and B are as defined
in claim 2;
and their physiologically tolerable salts.

4. The compound of the formula la as claimed in one or more of claims 1 to 3, in which:
R(1) and R(2)
independently of one another are hydrogen, CF₃ or alkyl having 1, 2 or 3 carbon atoms; or
R(1) and R(2)
together are an alkylene chain having 2, 3, 4, 5 or 6 carbon atoms;
R(3) is R(10)-CₙH₂ₙ;
R(10) is methyl or CF₃;
n is zero, 1 or 2;
R(4) is R(13)-CᵣH₂ᵣ,
where a CH₂ group of the group CᵣH₂ᵣ may be replaced by -O-, -CO-, -CO-O-, -O-CO-, -S-, -SO-, -SO₂- or -NR(14)-; R(14) is hydrogen or alkyl having 1, 2 or 3 carbon atoms;
R(13) is CH₃, CF₃, NR(15)R(16), phenyl or a nitrogen-containing heterocycle having 1, 2, 3, 4, 5, 6, 7, 8 or 9 carbon atoms,
where phenyl and the nitrogen-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15) and R(16) together are a chain of 4 or 5 methylene groups of which one CH₂ group may be replaced by -O-, -S-, -NH-, -N(methyl)- or -N(benzyl)-;
r is zero, 1, 2, 3, 4, 5, 6, 7 or 8;
R(5) is -Y-CₛH₂ₛ-R(18);
Y is -O-;
s is 1, 2, 3, 4, 5 or 6;
R(18) is hydrogen, CF₃, -COOR(21), NR(15a)R(16a), an unsubstituted nitrogen-containing heterocycle having 1, 2, 3, 4, 5, 6, 7, 8 or 9 carbon atoms, phenyl or thienyl,
where phenyl and thienyl are unsubstituted or substituted by 1 or 2 substituents selected from the group consisting of F, Cl, Br, I, CF₃, methyl, methoxy, sulfamoyl, methylsulfonyl and methylsulfonylamino;
R(15a) and R(16a)
together are a chain of 4 or 5 methylene groups of which one CH₂ group may be replaced by -O-, -S-, -NH-, -N(methyl)- or -N(benzyl)-;
R(21) is alkyl having 1, 2 or 3 carbon atoms;
R(6) is OH;
B is hydrogen;
or
R(6) and B
together are a bond; and their physiologically tolerable salts.

5. The compound of the formula la as claimed in one or more of claims 1 to 4, in which:
R(1) and R(2)
are methyl;
R(3) is methyl or ethyl;
R(4) is R(13)-CᵣH₂ᵣ,
where a CH₂ group of the group CᵣH₂ᵣ may be replaced by -O-, -CO-, -CO-O- or -O-CO;
R(13) is CH₃ or CF₃;
r is zero, 1, 2, 3, 4, 5, 6, 7 or 8;
R(5) is -Y-CₛH₂ₛ-R(18);
Y is -O-;
s is 1,2, 3, 4, 5 or 6;
R(18) is hydrogen, CF₃, -COOR(21), phenyl or thienyl,
where phenyl and thienyl are unsubstituted or substituted by 1 or 2 substituents selected from the group consisting of F, Cl, Br, I, CF₃, methyl or methoxy;
R(21) is alkyl having 1, 2 or 3 carbon atoms;
R(6) and B
together are a bond; and their physiologically tolerable salts.

6. The compound of the formula la as claimed in one or more of claims 1 to 4, in which:
R(1) and R(2)
are methyl;
R(3) is methyl or ethyl;
R(4) is R(13)-CᵣH₂ᵣ,
where a CH₂ group of the group CᵣH₂ᵣ may be replaced by -O-, -CO-, -CO-O- or -O-CO;
R(13) is CH₃ or CF₃;
r is zero, 1, 2, 3, 4, 5, 6, 7 or 8;
R(5) is -Y-CₛH₂ₛ-R(18);
Y is -O-;
s is 1, 2, 3, 4, 5 or 6;
R(18) is hydrogen, CF₃, phenyl or thienyl,
where phenyl and thienyl are unsubstituted or substituted by 1 or 2 substituents selected from the group consisting of F, Cl, Br, CF₃, methyl or methoxy;
R(6) is OH;
B is hydrogen; and their physiologically tolerable salts.

7. A process for preparing compounds of the formula I as claimed in one or more of claims 1 to 6, which comprises
a) reacting a compound of the formula II, 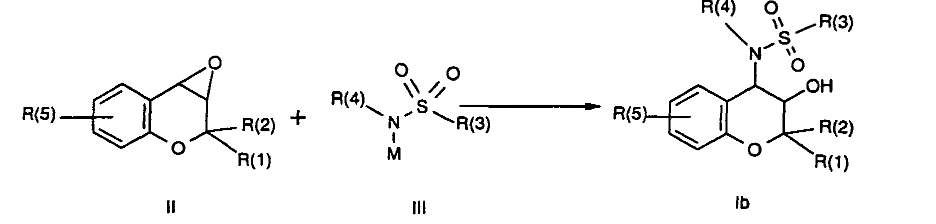 in which R(1), R(2) and R(5) are as defined in claims 1 to 6 with a sulfonamide of the formula III in which R(3) and R(4) are as defined in claims 1 to 6 and M is hydrogen or a metal equivalent or a trialkylsilyl radical to give a chromanol of the formula Ib;
or
b) reacting a compound of the formula Ib with an alkylating agent of the formula R(10d)-L or an acylating agent of the formula R(10d)-COL or an anhydride of the formula (R(10d)-CO)₂O, in which R(10d) is as defined in claims 1 to 4 and L is a nucleofugic leaving group, in a manner known per se in an alkylation or acylation reaction to give a compound of the formula Ic in which R(6) is OR(10d) or OCOR(10d); 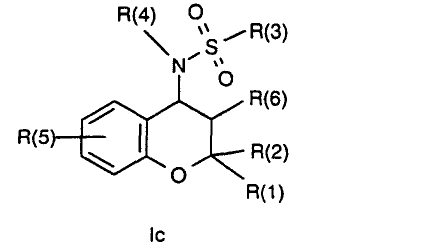 or
c) converting a compound of the formula Ic, 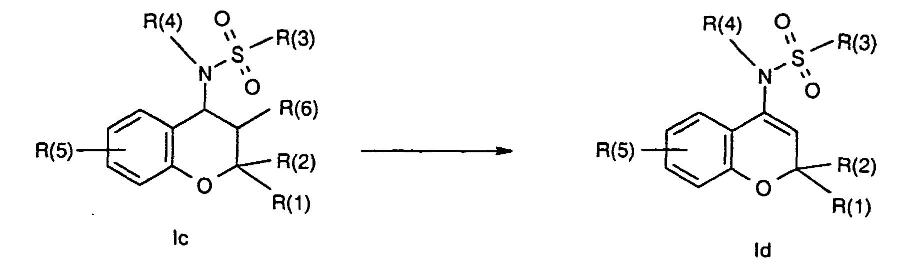 in which R(1), R(2), R(3), R(4) and R(5) are as defined in claims 1 to 6 and R(6) is OCOCH₃ in an elimination reaction to give a compound of the formula Id in which R(1), R(2), R(3), R(4) and R(5) are as defined in claims 1 to 6.

8. A compound of the formula I as claimed in one or more of claims 1 to 6 and its physiologically tolerable salts for use as a pharmaceutical.

9. A pharmaceutical preparation comprising an efficacious amount of at least one compound of the formula I as claimed in one or more of claims 1 to 6 and/or of a physiologically tolerable salt thereof as active compound, together with pharmaceutically acceptable excipients and additives and, if appropriate, additionally one or more other pharmacologically active compounds.

10. The use of a compound of the formula I as claimed in one or more of claims 1 to 6 and/or of a physiologically tolerable salt thereof for the production of a medicament having K⁺channel-blocking action for the therapy and prophylaxis of K⁺ channel-mediated diseases.

11. The use of a compound of the formula I as claimed in one or more of claims 1 to 6 and/or of a physiologically tolerable salt thereof for the production of a medicament for the inhibition of gastric acid secretion.

12. The use of a compound of the formula I as claimed in one or more of claims 1 to 6 and/or of a physiologically tolerable salt thereof for the production of a medicament for the therapy or prophylaxis of ulcers of the stomach or of the intestinal region.

13. The use of a compound of the formula I as claimed in one or more of claims 1 to 6 and/or of a physiologically tolerable salt thereof for the production of a medicament for the therapy or prophylaxis of reflux esophagitis.

14. The use of a compound of the formula I as claimed in one or more of claims 1 to 6 and/or of a physiologically tolerable salt thereof for the production of a medicament for the therapy or prophylaxis of diarrheal illnesses.

15. The use of a compound of the formula I as claimed in one or more of claims 1 to 6 and/or of a physiologically tolerable salt thereof for the production of a medicament for the therapy or prophylaxis of all types of arrhythmias, including atrial, ventricular and supraventricular arrhythmias.

16. The use of a compound of the formula I as claimed in one or more of claims 1 to 6 and/or of a physiologically tolerable salt thereof for the production of a medicament for the therapy or prophylaxis of cardiac arrhythmias which can be eliminated by action potential prolongation.

17. The use of a compound of the formula I as claimed in one or more of claims 1 to 6 and/or of a physiologically tolerable salt thereof for the production of a medicament for the therapy or prophylaxis of atrial fibrillation or atrial flutters.

18. The use of a compound of the formula I as claimed in one or more of claims 1 to 6 and/or of a physiologically tolerable salt thereof for the production of a medicament for the therapy or prophylaxis of reentry arrhythmias or for the prevention of sudden heart death as a result of ventricular fibrillation.

19. The use of a compound of the formula I as claimed in one or more of claims 1 to 6 and/or of a physiologically tolerable salt thereof for the production of a medicament for the therapy of cardiac insufficiency, in particular of congestive heart failure.

20. The use of a compound of the formula I as claimed in one or more of claims 1 to 6 and/or of a physiologically tolerable salt thereof for the production of a medicament for the inhibition of stimulated gastric acid secretion, for the therapy or prophylaxis of ulcers of the stomach or of the intestinal region, of reflux esophagitis, of diarrheal illnesses, for the therapy or prophylaxis of arrhythmias, including atrial, ventricular and supraventricular arrhythmias, atrial fibrillation and atrial flutters and reentry arrhythmias, or for the prevention of sudden heart death as a result of ventricular fibrillation.

21. A pharmaceutical preparation comprising an efficacious amount of at least one compound of the formula I as claimed in one or more of claims 1 to 6 and/or of a physiologically tolerable salt thereof and of a beta blocker as active compounds, together with pharmaceutically acceptable excipients and additives.

## Revendications

1. Composés de formule I, 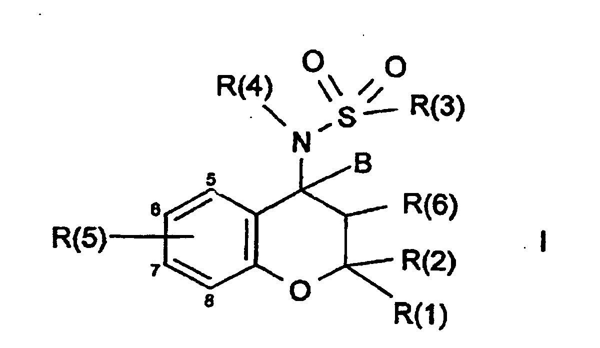 dans laquelle R(5) est lié en l'une des positions **caractérisées par** 5, 6, 7 et 8 et où :
R(1) et R(2) représentent,
chacun indépendamment l'un de l'autre, hydrogène, CF₃, C₂F₅, C₃F₇, un alkyle ayant 1, 2, 3, 4, 5 ou 6 atomes de C ou de phényle,
qui est non substitué ou substitué par 1 ou 2 substituants, choisis parmi le groupe composé de F, Cl, Br, I, CF₃, NO₂, CN, NH₂, OH, un méthyle, un éthyle, un méthoxy, un diméthylamino, un sulfamoyl, un méthylsulfonyle et un méthylsulfonylamino ;
ou
R(1) et R(2) représentent
ensemble une chaîne alkylène ayant 2, 3, 4, 5, 6, 7,
8, 9 ou 10 atomes de C ;
R(3) représente
R(10)-CₙH₂ₙ-NR(11) ou R(10)-CₙH₂ₙ-,
où un groupe CH₂ peut être remplacé dans les groupes CₙH₂ₙ par -O-, -CO-, -S-, -SO-, -SO₂- ou -NR(12a)- ;
R(12a) représente l'hydrogène, un méthyle ou un éthyle ;
R(10) représente l'hydrogène, un méthyle, un cycloalkyle ayant 3, 4, 5, 6, 7 ou 8 atomes de C, CF₃, C₂F₅ ou C₃F₇ ;
n étant égal à zéro, 1, 2, 3, 4, 5, 6, 7, 8, 9 ou 10 ;
R(11) représentant l'hydrogène ou un alkyle ayant 1, 2, 3, 4, 5 ou 6 atomes de C ;
ou
R(10) et R(11) représentent
ensemble une liaison dans la mesure où n n'est pas inférieur à 3 ;
ou
R(3) ensemble avec R(4) représentent
une chaîne alkylène ayant 3, 4, 5, 6, 7 ou 8 atomes de C,
où un groupe CH₂ de la chaîne alkylène peut être remplacé par -O-, -CO-, -S-, -SO-, -SO₂- ou -NR(12a)- ;
R(12a) représentant l'hydrogène, un méthyle ou un éthyle ;
R(4) représente
R(13)-CᵣH₂ᵣ,
où un groupe CH₂ du groupe -CᵣH₂ᵣ peut être remplacé par -O-, -CH=CH-, -C≡C-, -CO-, -CO-O-, -O-CO-, -S-, -SO-, -SO₂- ou -NR(14)- ;
R(14) représente l'hydrogène ou un alkyle ayant 1, 2 ou 3 atomes de C ;
R(13) représente
CH₃, CF₃, C₂F₅, C₃F₇, un cycloalkyle ayant 3, 4, 5, 6, 7 ou 8 atomes de C, NR(15)R(16), un phényle ou un hétérocycle contenant N ayant 1, 2, 3, 4, 5, 6, 7, 8 ou 9 atomes de C,
où le phényle et l'hétérocycle contenant N sont non substitués ou substitués par 1 ou 2 substituants, choisis parmi le groupe composé de F, Cl, Br, I, CF₃, NO₂, CN, NH₂, OH, méthyle, éthyle, méthoxy, diméthylamino, sulfamoyl, méthylsulfonyl et méthylsulfonylamino ;
R(15) et R(16) représentent
ensemble une chaîne de 4 ou 5 groupes méthylène dont un groupe CH₂ peut être remplacé par -O-, -S-, -NH-,-N(méthyl)- ou -N(benzyl)- ;
r est égal à
zéro, 1, 2, 3, 4, 5, 6, 7, 8, 9, 10, 11, 12, 13, 14, 15, 16, 17, 18, 19 ou 20 ;
R(5) représente
-Y-CₛH₂ₛ-R(18) ou un phényle,
qui est non substitué ou substitué par 1 ou 2 substituants choisis parmi le groupe composé de F, Cl, Br, I, CF₃, NO₂, CN, NH₂, OH, méthyle, éthyle, méthoxy, diméthylamino, sulfamoyl, méthylsulfonyl et méthylsulfonyl-amino ;
Y représente
-O-, -S- ou -NR(10c)- ;
R(10c) représente l'hydrogène ou un alkyle ayant 1, 2 ou 3 atomes de C ;
s est égal à
1, 2, 3, 4, 5, 6, 7 ou 8 ;
R(18) représente
l'hydrogène, CF₃, C₂F₅, C₃F₇, un cycloalkyle ayant 3, 4, 5, 6, 7 ou 8 atomes de C, -COOR(21), NR(15a)R(16a), un hétérocycle non substitué contenant N ayant 1, 2, 3, 4, 5, 6, 7, 8, ou 9 atomes de C, un phényle ou un thiényle,
où le phényle et le thiényle sont non substitués ou substitués par 1 ou 2 substituants, choisis parmi le groupe composé de F, Cl, Br, I, CF₃, méthyle, méthoxy, sulfamoyl, méthylsulfonyle et méthylsulfonylamino ;
R(15a) et R(16a) représentent
ensemble une chaîne de 4 ou 5 groupes méthylène dont un groupe CH₂ peut être remplacé par -O-, -S-, -NH-,-N(méthyl)- ou -N(benzyl)- ;
R(21) représente
l'hydrogène ou un alkyle ayant 1, 2 ou 3 atomes de C ;
R(6) représente
OR(10d) ou OCOR(10d) ;
R(10d) représente
l'hydrogène ou un alkyle ayant 1, 2 ou 3 atomes de C ;
B représente l'hydrogène ;
ou
R(6) et B représentent
ensemble une liaison ;
ainsi que leurs sels physiologiquement acceptables.

2. Composés de formule I selon la revendication 1, dans laquelle :
R(1) et R(2) représentent,
indépendamment l'un de l'autre, l'hydrogène, CF₃, C₂F₅, C₃F₇, ou un alkyle ayant 1, 2, 3, 4, 5 ou 6 atomes de C ;
ou
R(1) et R(2) représentent
ensemble une chaîne alkylène ayant 2, 3, 4, 5, 6, 7, 8, 9 ou 10 atomes de C,
R(3) représente
R(10)-CₙH₂ₙ ;
R(10) représente
un méthyle, CF₃, C₂F₅ ou C₃F₇ ;
n est égal à
zéro, 1, 2, 3, 4, 5 ou 6 ;
R(4) représente
R(13) -CᵣH₂ᵣ,
où un groupe CH₂ du groupe -CᵣH₂ᵣ peut être remplacé par -O-, -CH=CH-, -C≡C-, -CO-, -CO-O-, -O-CO-, -S-, -SO-, -SO₂- ou -NR(14)- ;
R(14) représente l'hydrogène ou un alkyle ayant 1, 2 ou 3 atomes de C ;
R(13) représente
CH₃, CF₃, C₂F₅, C₃F₇, un cycloalkyle ayant 3, 4, 5, 6, 7 ou 8 atomes de C, NR(15)R(16), un phényle ou un hétérocycle contenant N ayant 1, 2, 3, 4, 5, 6, 7, 8 ou 9 atomes de C,
où le phényle et l'hétérocycle contenant N sont non substitués ou substitués par 1 ou 2 substituants, choisis parmi le groupe composé de F, Cl, Br, I, CF₃, NO₂, CN, NH₂, OH, méthyle, éthyle, méthoxy, diméthylamino, sulfamoyl, méthylsulfonyl et méthylsulfonyl-amino ;
R(15) et R(16) représentent
ensemble une chaîne de 4 ou 5 groupes méthylène dont un groupe CH₂ peut être remplacé par -O-, -S-, -NH-,-N(méthyl)- ou -N(benzyl)- ;
r est égal à
zéro, 1, 2, 3, 4, 5, 6, 7, 8, 9, 10, 11 ou 12 ;
R(5) représente
-Y-CₛH₂ₛ-R(18) ou phényle,
qui est non substitué ou substitué par 1 ou 2 substituants choisis parmi le groupe composé de F, Cl, Br, I, CF₃, NO₂, CN, NH₂, OH, méthyle, éthyle, méthoxy, diméthylamino, sulfamoyl, méthylsulfonyl et méthylsulfonyl-amino ;
Y représente -O- ou -S- ;
s est égal à 1, 2, 3, 4, 5, 6, 7 ou 8 ;
R(18) représente
hydrogène, CF₃, C₂F₅, C₃F₇, un cycloalkyle ayant 3, 4, 5, 6, 7 ou 8 atomes de C, -COOR(21), NR(15a)R(16a), un hétérocycle non substitué contenant N ayant 1, 2, 3, 4, 5, 6, 7, 8, ou 9 atomes de C, un phényle ou un thiényle,
où le phényle et le thiényle sont non substitués ou substitués par 1 ou 2 substituants, choisis parmi le groupe composé de F, Cl, Br, I, CF₃, méthyle, méthoxy, sulfamoyl, méthylsulfonyle et méthylsulfonylamino ;
R(15a) et R(16a) représentent
ensemble une chaîne de 4 ou 5 groupes méthylène dont un groupe CH₂ peut être remplacé par -O-, -S-, -NH-,-N(méthyl)- ou -N(benzyl)- ;
R(21) représente un alkyle ayant 1, 2 ou 3 atomes de C ;
R(6) représente
OR(10d) ou OCOR(10d) ;
R(10d) représente hydrogène ou alkyle ayant 1, 2 ou 3 atomes de C ;
B représente l'hydrogène ;
ou
R(6) et B représentent ensemble une liaison ;
ainsi que leurs sels physiologiquement acceptables.

3. Composés de formule I selon les revendications 1 ou 2, dans laquelle R(5) est lié en la position **caractérisée par** 6, donc composés de formule Ia, 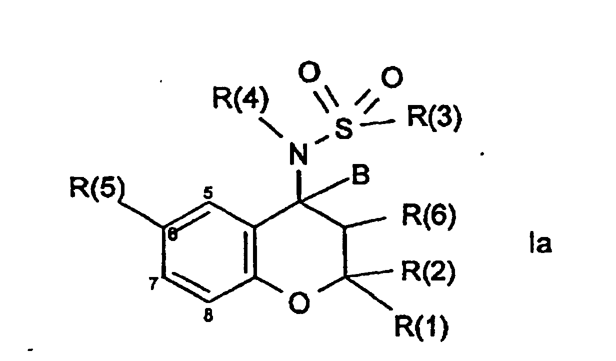 dans laquelle les radicaux R(1), R(2), R(3), R(4), R(5), R(6) et B ont les significations indiquées dans la revendication 2 ;
ainsi que leurs sels physiologiquement acceptables.

4. Composés de formule Ia selon l'une ou plusieurs des revendications 1 à 3, dans laquelle :
R(1) et R(2) représentent,
chacun indépendamment l'un de l'autre, l'hydrogène, CF₃, ou un alkyle ayant 1, 2 ou 3, atomes de C,
ou
R(1) et R(2) représentent
ensemble une chaîne alkylène ayant 2, 3, 4, 5 ou 6 atomes de C ;
R(3) représente
R(10) -CₙH₂ₙ-,
R(10) représente
méthyle ou CF₃ ;
n est égal à
zéro, 1 ou 2 ;
R(4) représente
R(13)-CᵣH₂ᵣ,
où un groupe CH₂ du groupe -CᵣH₂ᵣ peut être remplacé par -O-, -CO-, -CO-O-, -O-CO-, -S-, -SO-, -SO₂- ou-NR(14)- ;
R(14) représente hydrogène ou alkyle ayant 1, 2 ou 3 atomes de C ;
R(13) représente
CH₃, CF₃, NR(15)R(16), un phényle ou un hétérocycle contenant N ayant 1, 2, 3, 4, 5, 6, 7, 8 ou 9 atomes de C,
où le phényle et l'hétérocycle contenant N sont non substitués ou substitués par 1 ou 2 substituants, choisis parmi le groupe composé de F, Cl, Br, I, CF₃, NO₂, CN, NH₂, OH, méthyle, éthyle, méthoxy, diméthylamino, sulfamoyl, méthylsulfonyl et méthylsulfonyl-amino ;
R(15) et R(16) représentent
ensemble une chaîne de 4 ou 5 groupes méthylène dont un groupe CH₂ peut être remplacé par -O-, -S-, -NH-,-N(méthyl)- ou -N(benzyl)- ;
r est égal à
zéro, 1, 2, 3, 4, 5, 6, 7 ou 8 ;
R(5) représente
-Y-CₛH₂ₛ-R(18) ;
Y représente -O- ;
s est égal à 1, 2, 3, 4, 5 ou 6 ;
R(18) représente
hydrogène, CF₃, -COOR(21), NR(15a)R(16a), un hétérocycle non substitué contenant N ayant 1, 2, 3, 4, 5, 6, 7, 8, ou 9 atomes de C, un phényle ou un thiényle,
où le phényle et le thiényle sont non substitués ou substitués par 1 ou 2 substituants, choisis parmi le
groupe composé de F, Cl, Br, I, CF₃, méthyle, méthoxy, sulfamoyl, méthylsulfonyle et méthylsulfonylamino ;
R(15a) et R(16a) représentent
ensemble une chaîne de 4 ou 5 groupes méthylène dont un groupe CH₂ peut être remplacé par -O-, -S-, -NH-,-N(méthyl)- ou -N(benzyl)- ;
R(21) représente un alkyle ayant 1, 2 ou 3 atomes de C ;
R(6) représente OH ;
B représente l'hydrogène ;
ou
R(6) et B représentent ensemble une liaison ;
ainsi que leurs sels physiologiquement acceptables.

5. Composés selon la formule Ia selon l'une ou plusieurs des revendications 1 à 4, dans laquelle :
R(1) et R(2) représentent
méthyle ;
R(3) représente
un méthyle ou un éthyle ;
R(4) représente
R(13)-CᵣH₂ᵣ,
où un groupe CH₂ du groupe -CᵣH₂ᵣ peut être remplacé par -O-, -CO-, -CO-O-, -O-CO- ;
R(13) représente
CH₃ ou CF₃ ;
r est égal à
zéro, 1, 2, 3, 4, 5, 6, 7 ou 8 ;
R(5) représente
-Y-CₛH₂ₛ-R(18) ;
Y représente -O- ;
s est égal à 1, 2, 3, 4, 5 ou 6 ;
R(18) représente
l'hydrogène, CF₃, -COOR(21), un phényle ou un thiényle,
où le phényle et le thiényle sont non substitués ou substitués par 1 ou 2 substituants, choisis parmi le groupe composé de F, Cl, Br, CF₃, méthyle ou méthoxy ;
R(21) représentant alkyle avec 1, 2 ou 3 atomes de C ;
R(6) et B représentent ensemble une liaison ;
ainsi que leurs sels physiologiquement acceptables.

6. Composés selon la formule Ia selon l'une ou plusieurs des revendications 1 à 4, dans laquelle :
R(1) et R(2) représentent
un méthyle ;
R(3) représente
un méthyle ou un éthyle ;
R(4) représente
R(13)-CᵣH₂ᵣ,
où un groupe CH₂ du groupe -CᵣH₂ᵣ peut être remplacé par -O-, -CO-, -CO-O- ou -O-CO- ;
R(13) représente CH₃ ou CF₃ ;
r est égal à zéro, 1, 2, 3, 4, 5, 6, 7 ou 8 ;
R(5) représente -Y-CₛH₂ₛ-R(18) ;
Y représente -O- ;
s est égal à 1, 2, 3, 4, 5 ou 6 ;
R(18) représente
l'hydrogène, CF₃, un phényle ou un thiényle,
où le phényle et le thiényle sont non substitués ou substitués par 1 ou 2 substituants, choisis parmi le groupe composé de F, Cl, Br, CF₃, méthyle ou méthoxy ;
R(6) représente OH ;
B représente l'hydrogène
ainsi que leurs sels physiologiquement acceptables.

7. Procédé de préparation des composés de formule I selon l'une ou plusieurs des revendications 1 à 6, **caractérisé en ce que** l'on fait réagir
a) un composé de formule II, 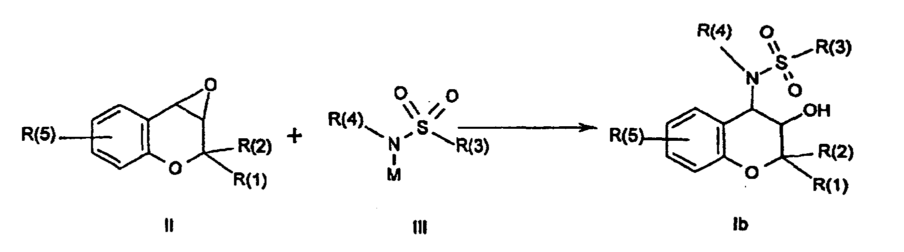 où R(1), R(2) et R(5) possèdent les significations indiquées dans les revendications 1 à 6, avec un sulfonamide de formule III, dans laquelle R(3) et R(4) ont les significations indiquées dans les revendications 1 à 6 et M représente hydrogène ou un équivalent métallique ou un radical trialkylsilyle, en un chromanol de formule Ib ;
ou **en ce que** l'on fait réagir
b) un composé de formule Ib avec un agent alkylant de formule R(10d)-L ou un agent acylant de formule R(10d)-COL ou un anhydride de formule (R(10d)-CO)₂O, où R(10d) possède les significations indiquées dans les revendications 1 à 4 et L représente un groupe fuyant nucléofuge, de manière connue au sens d'une réaction d'alkylation ou d'acylation, en un composé de formule Ic, dans laquelle R(6) représente OR(10d) ou OCOR(10d) ; 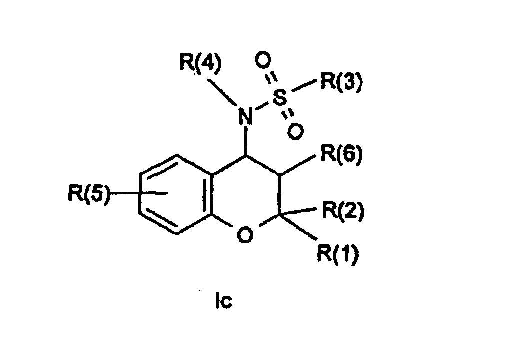 ou **en ce que** l'on fait réagir
c) un composé de formule Ic, 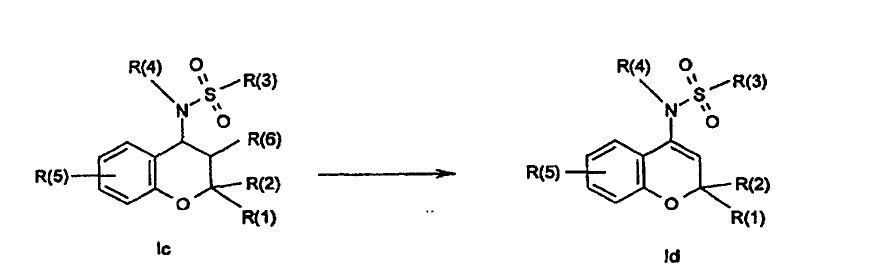
où R(1), R(2), R(3), R(4) et R(5) possèdent les significations indiquées dans les revendications 1 à 6, et R(6) représente OCOCH₃, au sens d'une réaction d'élimination, en un composé de formule Id, où R(1), R(2), R(3), R(4) et R(5) possèdent les significations indiquées dans les revendications 1 à 6.

8. Composés de formule I selon l'une ou plusieurs des revendications 1 à 6 et leurs sels physiologiquement acceptables destinés à être utilisés comme médicaments.

9. Préparation pharmaceutique contenant une quantité efficace d'au moins un composé de formule I selon l'une ou plusieurs des revendications 1 à 6 et/ou d'un sel physiologiquement acceptable de celui-ci comme principe actif, en compagnie d'excipients et additifs pharmaceutiquement acceptables et éventuellement encore un ou plusieurs autres principes actifs pharmacologiques.

10. Utilisation d'un composé de formule I selon l'une ou plusieurs des revendications 1 à 6 et/ou d'un sel physiologiquement acceptable de celui-ci pour la préparation d'un médicament ayant une action bloquant le canal K⁺ pour la thérapie et la prophylaxie des maladies à médiation par le canal K⁺.

11. Utilisation d'un composé de formule I selon l'une ou plusieurs des revendications 1 à 6 et/ou d'un sel physiologiquement acceptable de celui-ci pour la préparation d'un médicament destiné à inhiber la sécrétion des sucs gastriques.

12. Utilisation d'un composé de formule I selon l'une ou plusieurs des revendications 1 à 6 et/ou d'un sel physiologiquement acceptable de celui-ci pour la préparation d'un médicament pour la thérapie ou la prophylaxie des ulcères de l'estomac ou de la région intestinale.

13. Utilisation d'un composé de formule I selon l'une ou plusieurs des revendications 1 à 6 et/ou d'un sel physiologiquement acceptable de celui-ci pour la préparation d'un médicament pour la thérapie ou la prophylaxie de l'oesophagite de reflux.

14. Utilisation d'un composé de formule I selon l'une ou plusieurs des revendications 1 à 6 et/ou d'un sel physiologiquement acceptable de celui-ci pour la préparation d'un médicament pour la thérapie ou la prophylaxie des maladies diarrhéiques.

15. Utilisation d'un composé de formule I selon l'une ou plusieurs des revendications 1 à 6 et/ou d'un sel physiologiquement acceptable de celui-ci pour la préparation d'un médicament pour la thérapie ou la prophylaxie de tous les types d'arythmie, y compris les arythmies atriales, ventriculaires et supraventriculaires.

16. Utilisation d'un composé de formule I selon l'une ou plusieurs des revendications 1 à 6 et/ou d'un sel physiologiquement acceptable de celui-ci pour la préparation d'un médicament pour la thérapie ou la prophylaxie des troubles du rythme cardiaque pouvant être supprimés par la prolongation du potentiel d'action.

17. Utilisation d'un composé de formule I selon l'une ou plusieurs des revendications 1 à 6 et/ou d'un sel physiologiquement acceptable de celui-ci pour la préparation d'un médicament pour la thérapie ou la prophylaxie de la fibrillation atriale ou le flottement atrial.

18. Utilisation d'un composé de formule I selon l'une ou plusieurs des revendications 1 à 6 et/ou d'un sel physiologiquement acceptable de celui-ci pour la préparation d'un médicament pour la thérapie ou la prophylaxie de l'arythmie par réentrée ou pour empêcher l'arrêt soudain du coeur à la suite de fibrillations ventriculaires.

19. Utilisation d'un composé de formule I selon l'une ou plusieurs des revendications 1 à 6 et/ou d'un sel physiologiquement acceptable de celui-ci pour la préparation d'un médicament pour la thérapie de l'insuffisance cardiaque, en particulier de l'insuffisance cardiaque congestive (Congestive Heart Failure).

20. Utilisation d'un composé de formule I selon l'une ou plusieurs des revendications 1 à 6 et/ou d'un sel physiologiquement acceptable de celui-ci pour la préparation d'un médicament destiné à inhiber la sécrétion des sucs gastriques, pour la thérapie ou la prophylaxie des ulcères de l'estomac ou de la région intestinale, de l'oesophagite de reflux, des maladies diarrhéiques, pour la thérapie ou la prophylaxie des arythmies, y compris des arythmies atriales, ventriculaires, supraventriculaires, de la fibrillation atriale et du flottement atrial et des arythmies par réentrée ou pour empêcher l'arrêt soudain du coeur à la suite de fibrillations ventriculaires.

21. Préparation pharmaceutique contenant une quantité efficace d'au moins un composé de formule I selon l'une ou plusieurs des revendications 1 à 6 et/ou d'un sel physiologiquement acceptable de celui-ci ainsi que d'un bêta-bloquant comme principe actif, en compagnie d'excipients et additifs pharmaceutiquement acceptables.
